# EUROPEAN PATENT SPECIFICATION

(11) **EP 2 464 356 B1**
(45) Date of publication and mention of the grant of the patent: **06.05.2015**
(21) Application number: 10744560.3
(22) Date of filing: 12.08.2010
(51) Int. Cl.: A61K 31/5377, A61K 45/06, A61P 25/02, A61K 31/337, A61K 33/24

(54) **Sigma ligands for the prevention or treatment of pain induced by chemotherapy**
Sigma-Liganden zur Vorbeugung oder Behandlung von Schmerzen infolge von Chemotherapie
Ligands sigma pour la prévention ou le traitement de douleurs induites par la chimiothérapie

(30) Priority: 14.08.2009 EP 09382144
(43) Date of publication of application: 20.06.2012
(73) Proprietor: LABORATORIOS DEL DR. ESTEVE, S.A., 08041 Barcelona (ES)
(72) Inventor: BAEYENS-CABRERA, José Manuel, E-18014 Granada (ES); BUSCHMANN, Helmut, Heinrich, 52076 Aachen (Walheim) (DE); VELA HERNÁNDEZ, José Miguel, E-08028 Barcelona (ES); ZAMANILLO- CASTANEDO, Daniel, E-08041 Barcelona (ES); NIETO-LÓPEZ, Francisco-Rafael, E-18014 Barcelona (ES)
(74) Representative: ABG Patentes, S.L.
(86) International application number: PCT/EP2010/061720
(87) International publication number: WO 2011/018487

(56) References cited:
- WO-A1-2006/021462
- WO-A1-2007/098953
- ROH D-H ET AL: "Intrathecal injection of the [sigma]1 receptor antagonist BD1047 blocks both mechanical allodynia and increases in spinal NR1 expression during the induction phase of rodent neuropathic pain", ANESTHESIOLOGY, AMERICAN SOCIETY OF ANESTHESIOLOGISTS, PHILADELPHIA, PA, US, vol. 109, no. 5, 1 January 2008 (2008-01-01), pages 879-889, XP009128580, ISSN: 0003-3022
- PUENTE B D L ET AL: "Sigma-1 receptors regulate activity-induced spinal sensitization and neuropathic pain after peripheral nerve injury", PAIN 2009 ELSEVIER NLD, vol. 145, no. 3, 19 June 2009 (2009-06-19) , pages 294-303, XP002567072, ISSN: 0304-3959
- GAUCHAN PUNAM ET AL: "Mechanical allodynia induced by paclitaxel, oxaliplatin and vincristine: different effectiveness of gabapentin and different expression of voltage-dependent calcium channel alpha(2)delta-1 subunit.", BIOLOGICAL & PHARMACEUTICAL BULLETIN APR 2009, vol. 32, no. 4, April 2009 (2009-04), pages 732-734, XP002567073, ISSN: 0918-6158
- PACHARINSAK CHOLAWAT ET AL: "Animal models of cancer pain", COMPARATIVE MEDICINE (MEMPHIS), vol. 58, no. 3, June 2008 (2008-06), pages 220-233, XP002567074, ISSN: 1532-0820
- KIRCHMAIR RUDOLF ET AL: "Therapeutic angiogenesis inhibits or rescues chemotherapy-induced peripheral neuropathy: taxol- and thalidomide-induced injury of vasa nervorum is ameliorated by VEGF", MOLECULAR THERAPY, NATURE PUBLISHING GROUP, GB, vol. 15, no. 1, 1 January 2007 (2007-01-01), pages 69-75, XP002588572, ISSN: 1525-0024, DOI: DOI:10.1038/SJ.MT.6300019
- CHAUDHRY V ET AL: "77. Bortezomib and thalidomide-induced subacute demyelinating polyneuropathy", CLINICAL NEUROPHYSIOLOGY, ELSEVIER SCIENCE, IE, vol. 120, no. 2, 1 February 2009 (2009-02-01), page E111, XP025912377, ISSN: 1388-2457, DOI: DOI:10.1016/J.CLINPH.2008.10.095 [retrieved on 2009-02-01]

## Description

### FIELD OF THE INVENTION

The present invention relates to the use of a group of sigma receptor ligands in the prevention or treatment of pain resulting from chemotherapy. The present invention also refers to a combination of a sigma receptor ligand and a chemotherapeutic agent, and its use in the prevention or treatment of pain developing as a consequence of chemotherapy.

### BACKGROUND

The treatment of pain conditions is of great importance in medicine. There is currently a world-wide need for additional pain therapy. The pressing requirement for a specific treatment of pain conditions is documented in the large number of scientific works that have appeared recently in the field of applied analgesics.

PAIN is defined by the International Association for the Study of Pain (IASP) as "an unpleasant sensory and emotional experience associated with actual or potential tissue damage, or described in terms of such damage" (IASP, Classification of chronic pain, 2nd Edition, IASP Press (2002), 210). Although pain is always subjective, its causes or syndromes can be classified. Some of the most relevant pain subtypes are neuropathic pain, allodynia, hyperalgesia, and peripheral neuropathy.

On the other hand, cancer and its associated therapies are some of the biggest health concerns in the world. Chemotherapy, in combination with or as an alternative to surgery, is the method of choice in most cases for controlling or helping patients struck by carcinomas.

Chemotherapy is generally defined as the use of chemical substances to treat cancer, tumors or malign neoplasia and, in the sense of this invention, refers to the use of cytotoxic or cytostatic drugs, called chemotherapeutic drugs. In general it is a systemic treatment. Chemotherapy in cancer treatment consists of a personalized combination of potent chemotherapy drugs, designed to slow rapid cancer tumor growth, shrink tumors, kill cancer cells, and prevent the spread of cancer. The chemotherapeutic drugs prevent cells from replicating in the typical, out-of-control manner in which cancer cells divide.

Peripheral neurotoxicity is a clinically significant complication of cancer chemotherapy. For several of the most effective drugs (e.g. taxanes, vinca alkaloids, cisplatin, bortezomib, thalidomide and lenolidamide), neurotoxicity is dose-limiting and sometimes forces the termination of otherwise successful therapy (Polomano and Bennett, Pain Med., 2001, 2(1), 8-14; Park et al., Curr. Med. Chem, 2008,15(29), 3081-94). Since these drugs are the treatment of choice for a multitude of hematologic malignancies and solid tumours, hundred of thousands of patients are affected each year. Sensory abnormalities from antineoplastic-evoked neurotoxicity range from mild paresthesiae or dysesthesiae in many patients to a chronic painful peripheral neuropathy in some (Quasthoff and Hartung, J. Neurol., 2002, 249(1), 9-17). The occurrence and severity of the neuropathy is dependent on single dose intensity, duration of treatment, cumulative dose, prior or concurrent treatment with other neuropathic drugs and co-existing conditions such as diabetes and alcohol abuse (Alberts et al., Anticancer Drugs, 1995, 6(3), 369-83; Postma et al., Ann. Oncol., 1995, 6(5), 489-94; Forsyth et al., J. Neurooncol., 1997, 35(1), 47-53; Quasthoff and Hartung, J. Neurol., 2002, 249(1), 9-17). It is known in the art that peripheral neuropathy accompanied by neuropathic pain, allodynia and hyperalgesia develop in a considerable number of cases as a result of chemotherapy. These are very specific symptoms arising from the neurotoxicity of the chemotherapeutic drug. The treatment of these symptoms is crucial for preserving the quality of life of the afflicted patients (Mielke et al., Eur. J. Cancer, 2006, 42(1), 24-30; Park et al., Curr. Med. Chem., 2008, 15(29), 3081-94; Argyriou et al., Blood, 2008, 112(5), 1593-9). Unfortunately, an effective treatment for chemotherapy-induced peripheral neuropathy has yet to be found (Wolf et al., Eur. J. Cancer, 2008, 44(11), 1507-15).

Therefore, there is a need to provide a new form of prevention and treatment for pain, and especially for neuropathic pain, allodynia, hyperalgesia and peripheral neuropathy, developing after chemotherapy.

### BRIEF DESCRIPTION OF THE INVENTION

The inventors of the present invention have surprisingly found and demonstrated that the administration of some specific sigma receptor ligands is highly effective for preventing or treating the pain developing as a consequence of chemotherapy. They are particularly useful when the pain is neuropathic pain, allodynia or hyperalgesia. Sigma receptor ligands are highly effective for treating chemotherapy-induced neuropathic pain when administered after the antineoplastic drug, when pain has already developed. Even more surprisingly, this invention demonstrates that the co-administration of these sigma receptor ligands and a chemotherapeutic drug prevents the development of pain that would normally develop after chemotherapy. Therefore, sigma receptor ligands are effective both for the treatment (sigma receptor ligand administered after the chemotherapeutic drug, when pain has developed) and the prevention (co-administration of sigma receptor ligand and the chemotherapeutic drug) of chemotherapy-induced neuropathic pain.

Therefore, one aspect of the present invention relates to a combination of at least one sigma receptor ligand and at least one chemotherapeutic drug for simultaneous, separate or sequential administration, wherein the sigma ligand has the general formula (I): 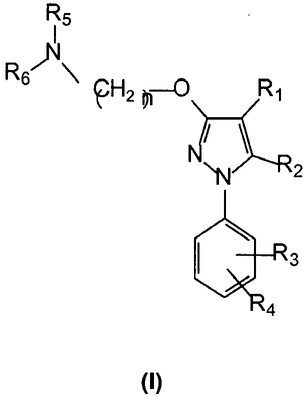 wherein
R₁ is selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic heterocyclyl, substituted or unsubstituted aromatic heterocyclyl, substituted or unsubstituted heterocyclylalkyl, -COR₈, -C(O)OR₈,-C(O)NR₈R₉, -CH=NR₈, -CN, -OR₈, -OC(O)R₈, -S(O)ₜ-R₈, -NR₈R₉, -NR₈C(O)R₉,-NO₂, -N=CR₈R₉, and halogen;
**R₂** is selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, -C(O)NR₈R₉, -CH=NR₈, -CN,-OR₈, -OC(O)R₈, -S(O)ₜ-R₈, -NR₈R₉, -NR₈C(O)R₉, -NO₂, -N=CR₈R₉, and halogen;
**R₃** and **R₄** are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈,-C(O)NR₈R₉, -CH=NR₈, -CN, -OR₈, -OC(O)R₈, -S(O)ₜ-R₈ , -NR₈R₉, -NR₈C(O)R₉,-NO₂, -N=CR₈R₉, and halogen, or together they form an optionally substituted fused ring system;
**R₅** and **R₆** are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈,-C(O)NR₈R₉, -CH=NR₈, -CN, -OR₈, -OC(O)R₈, -S(O)ₜ-R₈, -NR₈R₉, -NR₈C(O)R₉,-NO₂, -N=CR₈R₉, and halogen, or together form, with the nitrogen atom to which they are attached, a substituted or unsubstituted, aromatic or non-aromatic heterocyclyl group;
n is selected from 1, 2, 3, 4, 5, 6, 7 and 8;
**t** is 1,2 or 3;
**R₈** and **R₉** are each independently selected from hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer, prodrug or solvate thereof and the chemotherapeutic drug is selected from the group formed by taxanes, vinca alkaloids drugs derived from platinum and thelidomide.

In another aspect, the present invention relates to a compound of formula (I) as defined above, or a pharmaceutically acceptable salt, isomer, prodrug or solvate thereof, for its use in the prevention or treatment of pain induced by chemotherapy wherein the chemotherapeutic drug is as defined herein before.

Another aspect of this invention refers to a combination as defined above for its use in the manufacture of a medicament.

Another aspect of the invention relates to a combination as defined above for its use in the prevention or treatment of pain induced by chemotherapy.

Another aspect of the disclosure is a method of treatment of a patient suffering from pain induced by chemotherapy, or likely to suffer pain as a result of a chemotherapeutic treatment, which comprises administering to the patient in need of such a treatment or prophylaxis a therapeutically effective amount of a sigma ligand of formula (I) as defined above.

These aspects and preferred embodiments thereof are additionally also defined in the claims.

### BRIEF DESCRIPTION OF THE FIGURES

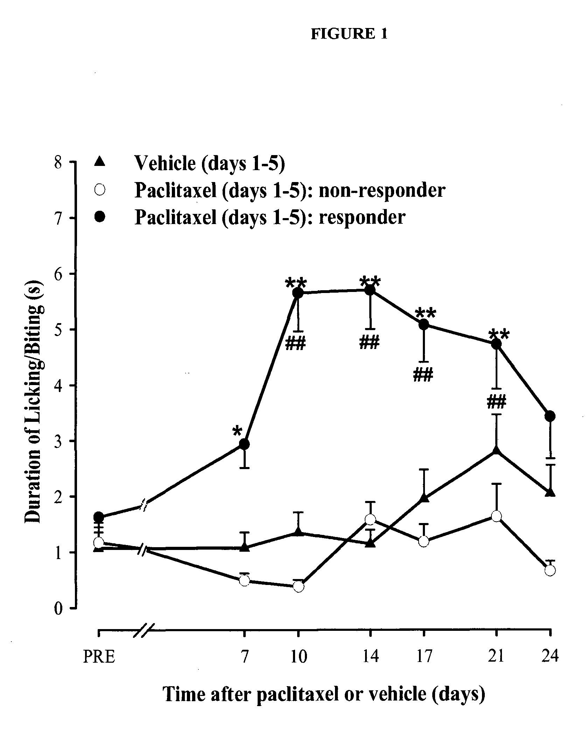

**Fig. 1****:** Time-course of paclitaxel induced cold-allodynia in mice. Each point and vertical line represents the mean ± S.E.M. of the values obtained in at least 12 mice. Statistically significant differences between the values of paclitaxel- and vehicle-treated groups: * *p* < 0.05; ** *p* < 0.01; and between the values on pre-treatment day and the days after treatment: # *p* < 0.05; ## *p* < 0.01 (two-way repeated measures ANOVA followed by Newman-Keuls test).

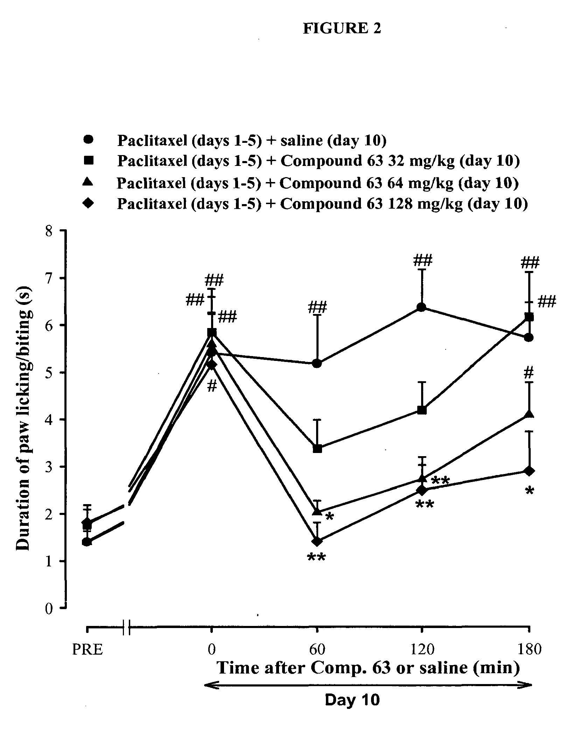

**Fig. 2****:** Time-course of the effect on the duration of hind paw licking/biting (acetone test) of a single s.c. injection of Compound 63 (32, 64 or 128 mg/kg) or saline in mice on day 10 after administration of paclitaxel. Each point and vertical line represents the mean ± S.E.M. of the values obtained in 14-22 animals. Statistically significant differences between the compound 63 and saline-treated groups on the same day after treatment: * *p* < 0.05; ** *p* < 0.01; and between the values obtained in the pretreatment day and in day 10 at different times after drug or saline administration: # *p* < 0.05; ## *p* < 0.01 (two-way repeated measures ANOVA followed by Newman-Keuls test).

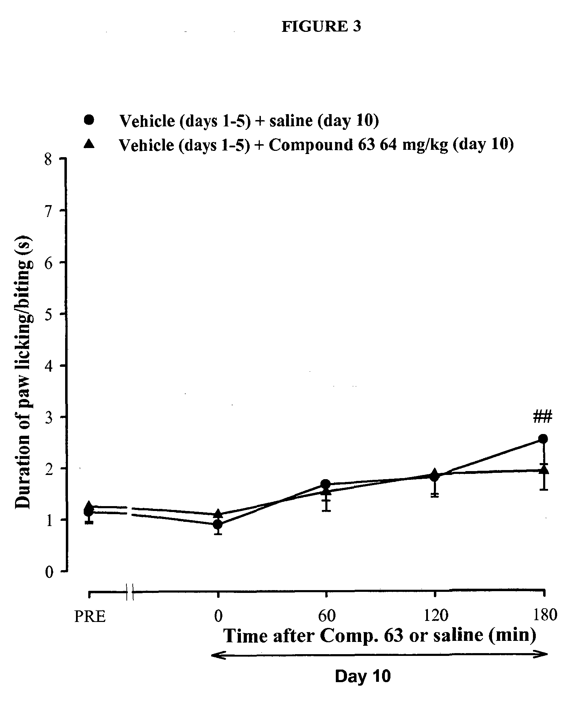

**Fig. 3****:** Time-course of the effect on the duration of hind paw licking/biting (acetone test) of a single s.c. injection of compound 63 (64 mg/kg) or saline in mice on day 10 after administration of paclitaxel-vehicle. Each point and vertical line represents the mean ± S.E.M. of the values obtained in 11-14 animals. No statistically significant differences between the two groups were observed at any observation time; statistically significant differences between the values obtained in the pretreatment day and in day 10 at different times after drug or saline administration: ## p < 0.01 (two-way repeated measures ANOVA followed by Newman-Keuls test).

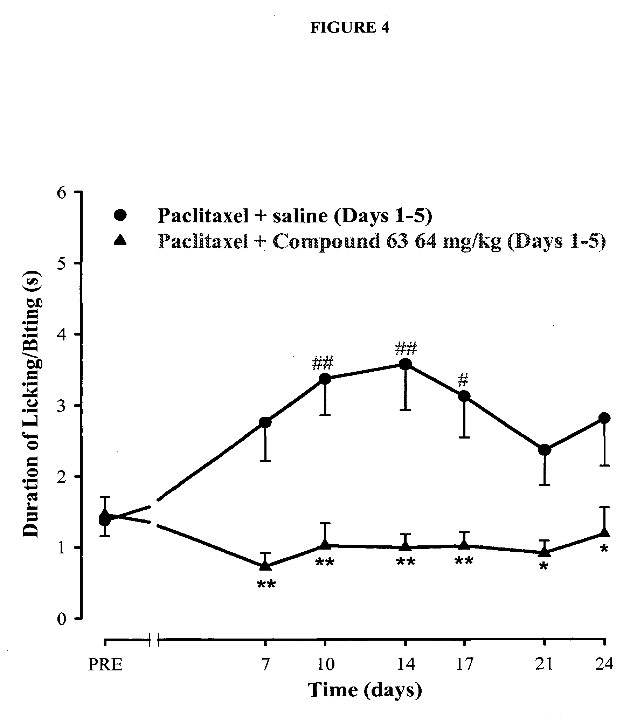

**Fig. 4****:** Time-course of the effect of coadministration of paclitaxel + compound 63 (64 mg/kg) and paclitaxel + saline on the duration of hind paw licking/biting in the acetone test. Each point and vertical line represents the mean ± S.E.M. of the values obtained in 25-35 animals. Statistically significant differences in comparison to paclitaxel + saline: * *p* < 0.05, ** *p* < 0.01; and between the values on the pretreatment day and the days after treatment: # *p* < 0.05, ## *p* < 0.01 (two-way repeated measures ANOVA followed by Newman-Keuls test).

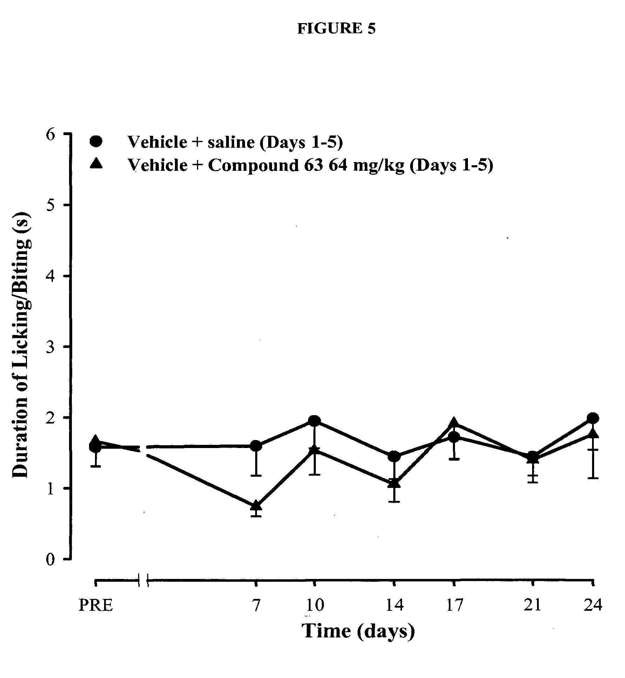

**Fig. 5****:** Time-course of the effect on the duration of hind paw licking/biting (acetone test) of coadministration of paclitaxel vehicle + compound 63 (64 mg/kg) and paclitaxel vehicle + saline. Each point and vertical line represents the mean ± S.E.M. of the values obtained in 15-20 animals. No statistically significant differences between the two groups were observed at any observation time, or in comparison to their own pretreatment day values (two-way repeated measures ANOVA).

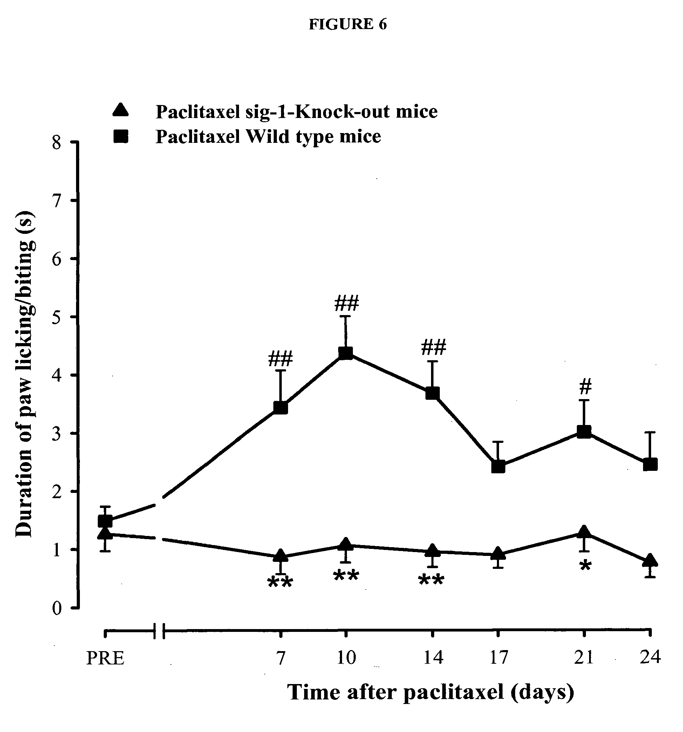

**Fig. 6****:** Time-course of paclitaxel-induced cold allodynia in sigma-1 receptors knock-out and wild type mice. Each point and vertical line represents the mean ± S.E.M. of the values obtained in 48 (wild type) and 21 (knock-out) animals. Statistically significant differences between the sigma-1 receptor knock-out and wild type mice on the same day after treatment: ** *p* < 0.01; and between the values on the pretreatment day and the days after treatment: # *p* < 0.05; ## *p* < 0.01 (two-way repeated measures ANOVA followed by Newman-Keuls test).

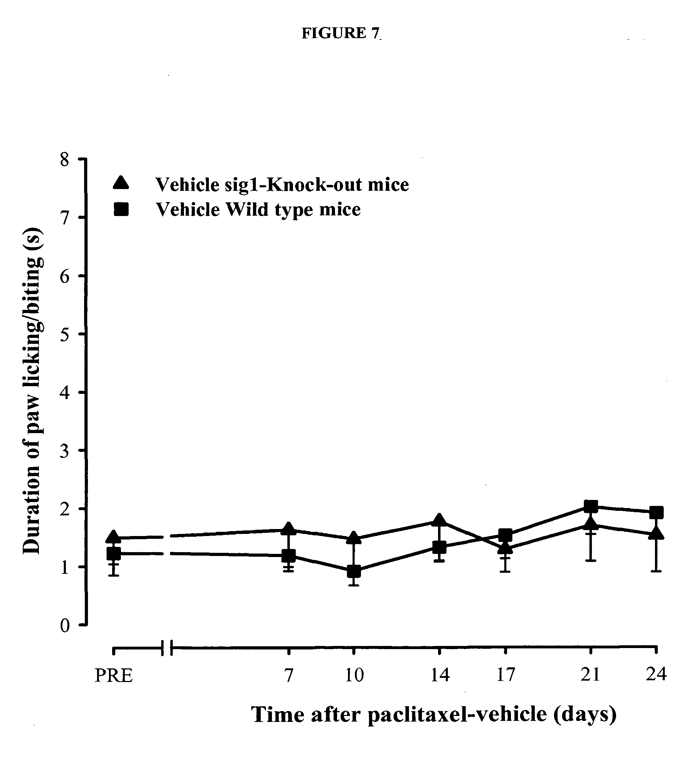

**Fig. 7****:** Time-course of the effect of paclitaxel-vehicle on the duration of hind paw licking/biting in the acetone test in sigma-1 receptor knock-out and wild type mice. Each point and vertical line represents the mean ± S.E.M. of the values obtained in 31 (wild type) and 15 (knock-out) animals No statistically significant differences between the two groups were found at any observation time or in comparison to their own pretreatment day values and the days after treatment (two-way repeated measures ANOVA).

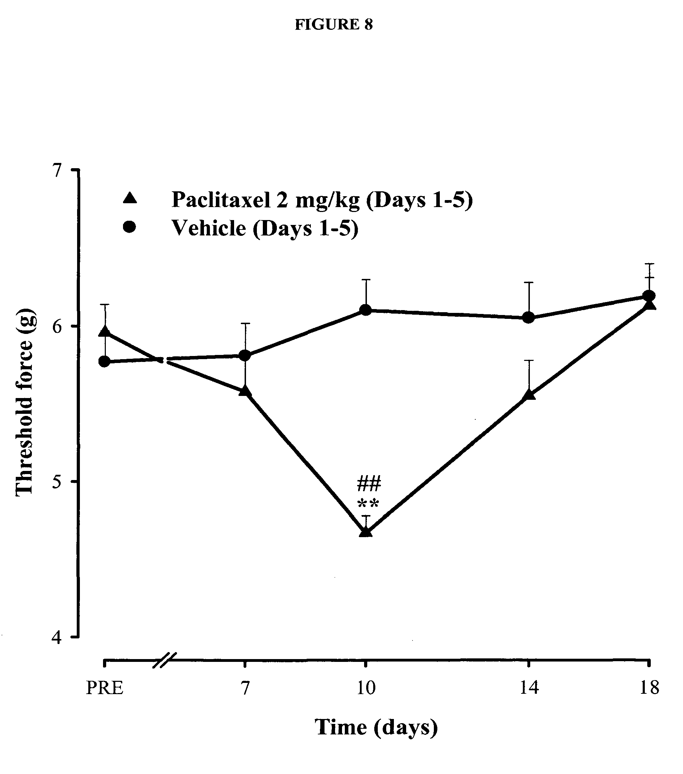

**Fig. 8****:** Time-course of paclitaxel-induced mechanical allodynia in mice. Each point and vertical line represents the mean ± S.E.M. of the values obtained in 32-34 animals. Statistically significant differences between the paclitaxel- and vehicle-treated groups on the same day after treatment: ** *p* < 0.01; and between the values on the pretreatment day and the days after treatment: ## *p* < 0.01 (two-way repeated measures ANOVA followed by Newman-Keuls test).

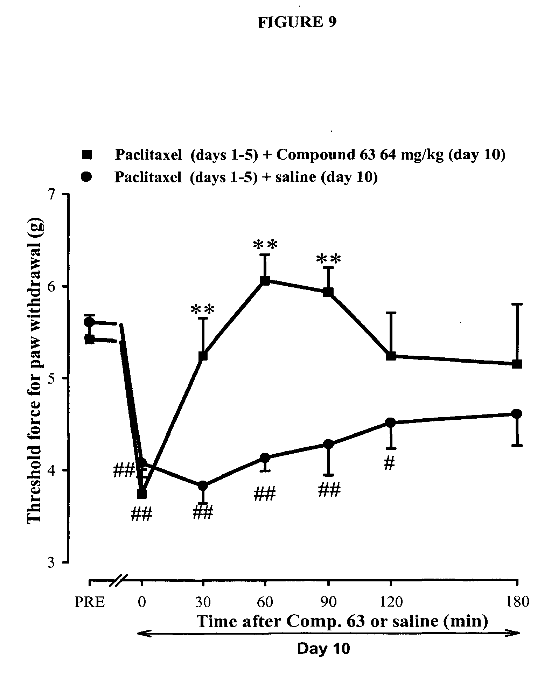

**Fig. 9****:** Time-course of the effect on the threshold force for hind paw withdrawal of a single s.c. injection of compound 63 (64 mg/kg) or saline in mice on day 10 after administration of paclitaxel. Each point and vertical line represents the mean ± S.E.M. of the values obtained in 10-14 animals. Statistically significant differences among the compound 63 and saline-treated groups at the same time after treatment: ** *p* < 0.01; and between the values obtained in the pretreatment day and in day 10 at different times after drug or saline administration: # *p* < 0.05; ## *p* < 0.01 (two-way repeated measures ANOVA followed by Newman-Keuls test).

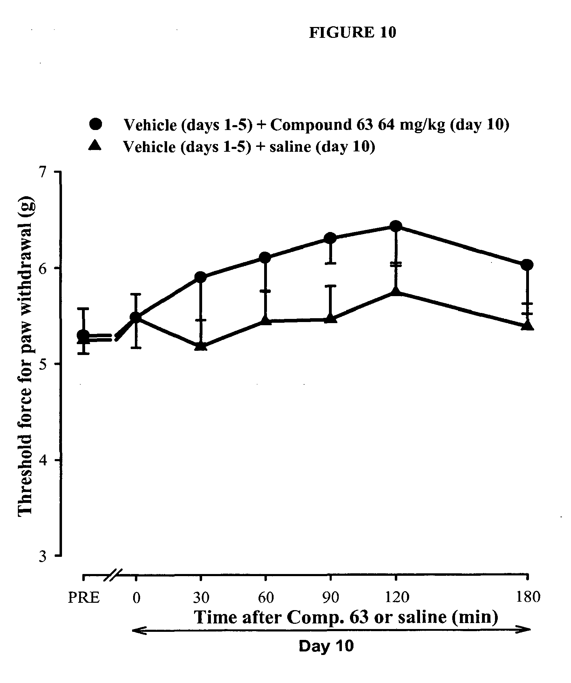

**Fig. 10****:** Time-course of the effect on the threshold force for hind paw withdrawal of a single s.c. injection of compound 63 (64 mg/kg) or saline in mice on day 10 after administration of paclitaxel-vehicle. Each point and vertical line represents the mean ± S.E.M. of the values obtained in 7-10 animals. No statistically significant differences among the two groups were observed at any observation time, or within each group in comparison to their own pretreatment day values (two-way repeated measures ANOVA).

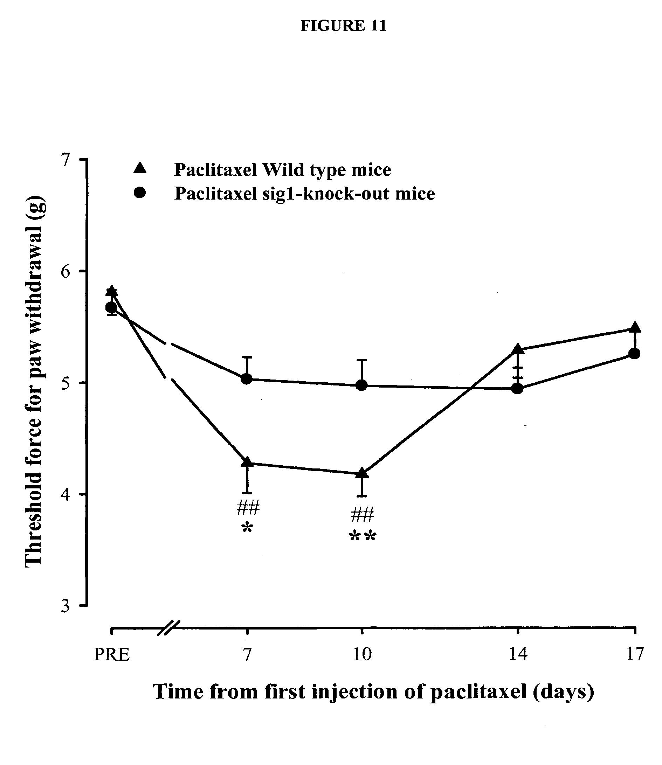

**Fig. 11****:** Time-course of paclitaxel-induced mechanical allodynia in sigma-1 receptor knockout and wild type mice. Each point and vertical line represents the mean ± S.E.M. of the values obtained in 18 (wild type) and 20 (knockout) animals. Statistically significant differences between the sigma-1 receptor knockout and wild type mice on the same day after treatment: * *p* < 0.05; ** *p* < 0.01; and between the values on the pretreatment day and the days after treatment: ## *p* < 0.01 (two-way repeated measures ANOVA followed by Newman-Keuls test).

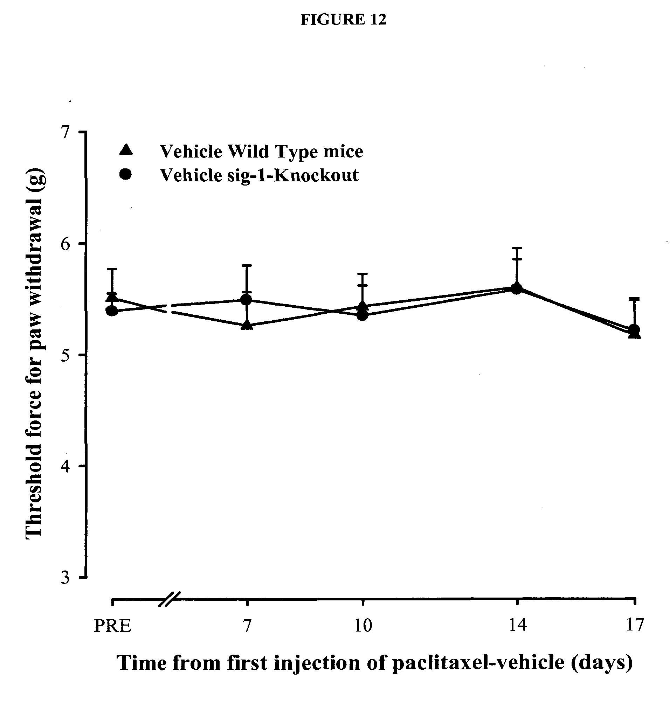

**Fig. 12****:** Time-course of the effect of paclitaxel-vehicle on the threshold force for hind paw withdrawal in sigma-1 receptor knockout and wild type mice. Each point and vertical line represents the mean ± S.E.M. of the values obtained in 12 animals. No statistically significant differences between the two groups were found at any observation time or in comparison to their own pretreatment day values and the days after treatment (two-way repeated measures ANOVA).

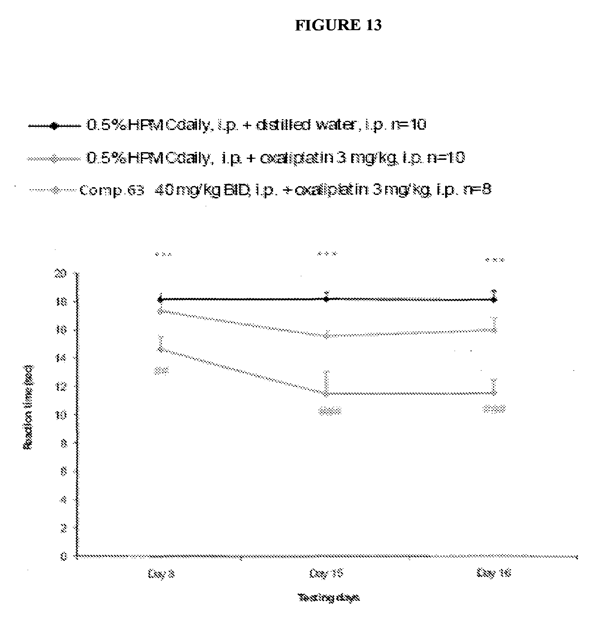

**Fig. 13****:** Preventive (coadministration paradigm) antiallodynic effects of compound 63 in a chronic model of oxaliplatin-induced neuropathy in rats (*Acetone Test* / *Reaction Time*). Results are expressed as the reaction time of paw withdrawal (mean ± SEM) in sec / experimental group / testing day, calculated from individual reaction time (mean values of the reaction time obtained for both hindpaws). ##; ### : p < 0.01 and p < 0.001 as compared to the HPMC/distilled water-treated group, Bonferroni's test after significant Two way ANOVA on Ranks. *** : p < 0.001 as compared to the 0.5% HPMC / Oxaliplatin-treated group, Bonferroni's test after significant Two way ANOVA on Ranks.

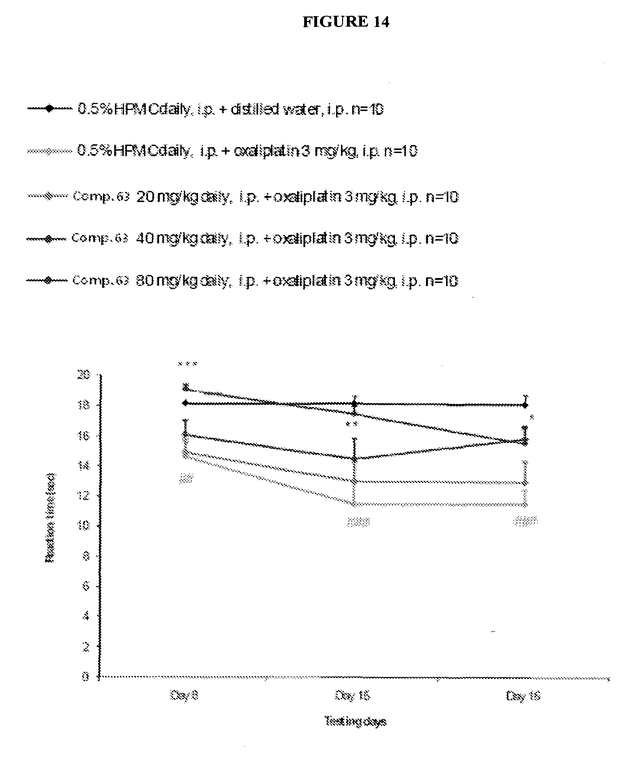

**Fig. 14****:** Antiallodynic effects of compound 63 (administration after oxaliplatin) in a chronic model of oxaliplatin-induced neuropathy in rats (*Acetone Test*/*Reaction Time*). Results are expressed as the reaction time of paw withdrawal (mean ± SEM) in sec / experimental group / testing day, calculated from individual reaction time (mean values of the reaction time obtained for both hindpaws). ##; ### : p < 0.01 and p < 0.001 as compared to the HPMC/distilled water-treated group, Bonferroni's test after significant Two way ANOVA on Ranks. ***, ** and * : p < 0.001, p < 0.01 and p < 0.05 as compared to the 0.5% HPMC / Oxaliplatin-treated group, Bonferroni's test after significant Two way ANOVA on Ranks.

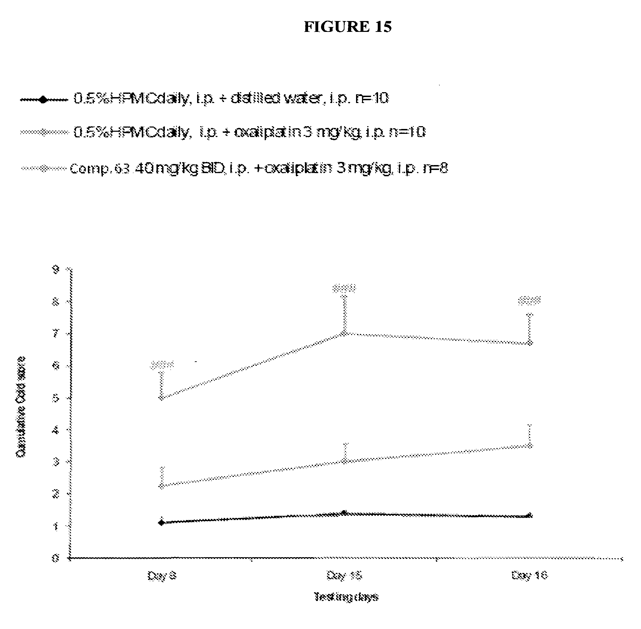

**Fig. 15****:** Preventive (coadministration paradigm) antiallodynic effects of compound 63 in a chronic model of oxaliplatin-induced neuropathy in rats *(Acetone TestlCumulative Cold Score).* Results (Cumulative Cold Score) are expressed as the mean ± S.E.M. of the sum of the 6 scores obtained for both hindpaws / experimental group / testing day. ### : p < 0.001 as compared to the HPMC/distilled water-treated group, Bonferroni's test after significant Two way ANOVA on Ranks. *** : p < 0.001 as compared to the 0.5% HPMC / Oxaliplatin-treated group, Bonferroni's test after significant Two way ANOVA on Ranks.

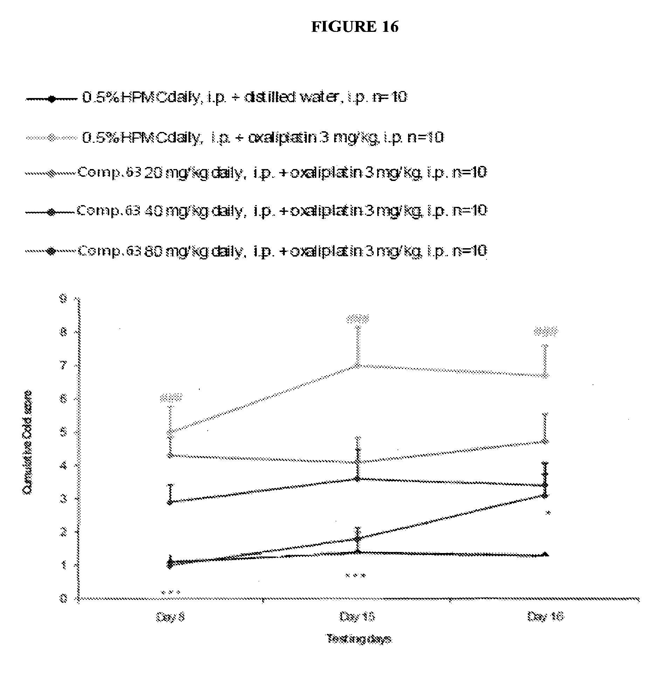

**Fig. 16****:** Antiallodynic effects of compound 63 (administration after oxaliplatin) in a chronic model of oxaliplatin-induced neuropathy in rats (*Acetone Test* / *Cumulative Cold Score*). Results (Cumulative Cold Score) are expressed as the mean ± S.E.M. of the sum of the 6 scores obtained for both hindpaws / experimental group / testing day. ### : p < 0.001 as compared to the HPMC/distilled water-treated group, Bonferroni's test after significant Two way ANOVA on Ranks. *** and *: p < 0.001 and p < 0.05 as compared to the 0.5% HPMC / Oxaliplatin-treated group, Bonferroni's test after significant Two way ANOVA on Ranks.

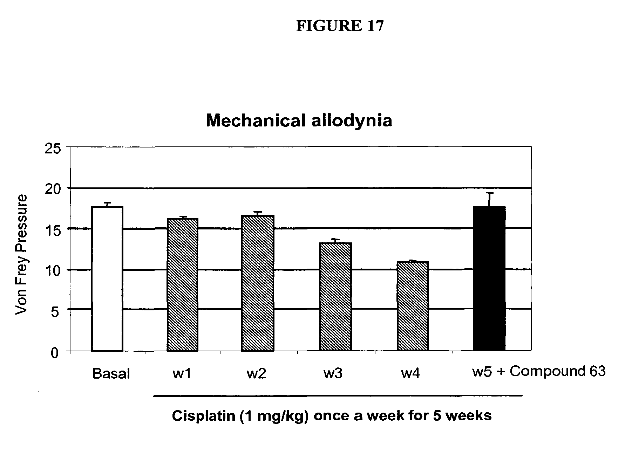

**Fig. 17****:** Acute treatment effect of compound 63 in a chronic model of cisplatin-induced neuropathy in rats. Cisplatin (1 mg/kg) was administered once a week for 5 weeks and compound 63 (64 mg/kg) was administered on week 5 (50 minutes before the test).

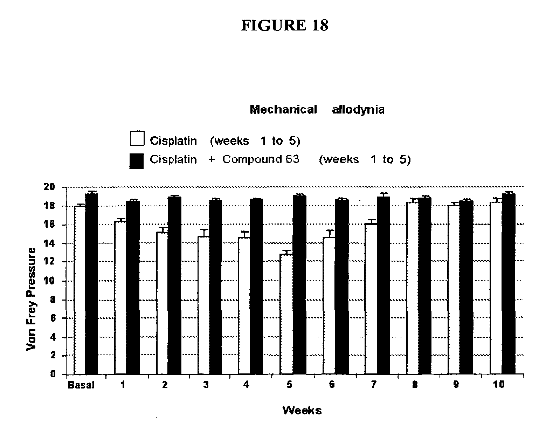

**Fig. 18****:** Preventive effect of compound 63 in a chronic model of cisplatin-induced neuropathy in rats. Cisplatin (1 mg/kg) and compound 63 (25 mg/kg) were coadministered one day per week for 5 weeks.

### DETAILED DESCRIPTION OF THE INVENTION

In the context of the present invention, the following terms have the meaning detailed below.

"Alkyl" refers to a straight or branched hydrocarbon chain radical consisting of 1 to 12 carbons, containing no unsaturation, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as aryl, halo, hydroxy, alkoxy, carboxy, cyano, carbonyl, acyl, alkoxycarbonyl, amino, nitro, mercapto, alkylthio, etc. If substituted by aryl, it corresponds to an "Arylalkyl" radical, such as benzyl and phenethyl. Preferred alkyl radicals have from 1-6 carbon atoms.

"Alkenyl" refers to an alkyl radical consisting of 2 to 12 carbons and having one or more unsaturated bonds.

"Cycloalkyl" refers to a stable 3-to 10-membered monocyclic or bicyclic radical which is saturated or partially saturated, and which consist solely of carbon and hydrogen atoms, such as cyclohexyl or adamantyl. Unless otherwise stated specifically in the specification, the term"cycloalkyl" is meant to include cycloalkyl radicals which are optionally substituted by one or more substituents such as alkyl, halo, hydroxy, amino, cyano, nitro, alkoxy, carboxy, alkoxycarbonyl, etc.

"Aryl" refers to single and multiple aromatic ring radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, fenanthryl or anthracyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

"Heterocyclyl" refers to a stable 3-to 15 membered ring radical which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. It may be aromatic or not aromatic. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized ; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc.

"Alkoxy" refers to a radical of the formula -ORₐ where Rₐ is an alkyl radical as defined above, e. g., methoxy, ethoxy, propoxy, etc.

"Amino" refers to a radical of the formula -NH₂, -NHRₐ or -NRₐR_{b}, optionally quaternized, wherein Rₐ and R_{b} are independently an alkyl radical as defined above e.g., methylamino, ethylamino, dimethylamino, diethylamino, propylamino, etc.

"Halo" or "hal" refers to bromo, chloro, iodo or fluoro.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C₁₋₆ alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "salt" must be understood as any form of an active compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts".

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used on humans and/or mammals. Salts with alkali and alkali earth metals are preferred particularly, as well as those formed with ammonium cations (NH₄⁺). Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of the active compound in accordance with the invention in which said compound is bonded by a non-covalent bond to another molecule (normally a polar solvent), including especially hydrates and alcoholates, like for example, methanolate. A preferred solvate is the hydrate.

Any compound that is a prodrug of a compound of formula I is also within the scope of the invention. The term "prodrug" is used in its broadest sense and encompasses those derivatives that are converted in vivo to the compounds of the invention. Examples of prodrugs include, but are not limited to, derivatives and metabolites of the compounds of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

As used herein, the terms "treat", "treating" and "treatment" include the eradication, removal, reversion, alleviation, modification, or control of pain induced by chemotherapy, after the pain onset.

As used herein, the terms "prevention", "preventing", "preventive" "prevent" and prophylaxis refers to the capacity of a therapeutic to avoid, minimize or difficult the onset or development of a disease or condition before its onset, in this case pain induced by chemotherapy.

As used herein the term "chemotherapy" or "chemotherapeutic drug" refers broadly to the use of chemical drugs for the treatment of cancer, tumors or malign neoplasia.

"Developing as a consequence of chemotherapy" according to this invention is defined as: a) developing after or with at the beginning of chemotherapy and b) thus coinciding with or following the use of a chemotherapeutic drug. Therefore, the symptom to be treated is likely to be caused by or is due to the toxicity, cytotoxicity or especially, the peripheral neurotoxicity, of the chemotherapeutic drug.

In a preferred embodiment, R₁ in compounds of formula (I) is selected from H, -COR₈, and substituted or unsubstituted alkyl. More preferably, R₁ is selected from H, methyl and acetyl. A more preferred embodiment is when R₁ is H.

In another preferred embodiment, R₂ represents H or alkyl, more preferably methyl.

In yet another preferred embodiment of the invention, R₃ and R₄ are situated in the meta and para positions of the phenyl group, and preferably, they are selected independently from halogen and substituted or unsubstituted alkyl.

In an especially preferred embodiment of the invention, both R₃ and R₄ together with the phenyl group form an optionally substituted fused ring system, more preferably, a naphthyl ring system.

Also, embodiments where n is selected from 2, 3, 4 are preferred in the context of the present invention, more preferably n is 2.

Finally, in another embodiment it is preferred that R₅ and R₆ are, each independently, C₁₋₆alkyl, or together with the nitrogen atom to which they are attached form a morpholinyl, piperidinyl, or pyrrolidinyl group. More preferably, R₅ and R₆ together form a morpholine-4-yl group.

In preferred variants of the invention, the combination of the invention encompasses a sigma ligand of formula (I) selected from:
[1] 4-{2-(1-(3,4-dichlorophenyl)-5-methyl-1 H pyrazol-3-yloxy)ethyl}morpholine
[2] 2-[1-(3,4-Dichlorophenyl)-5-methyl-1H-pyrazol-3-yloxy]-N,N-diethylethanamine
[3] 1-(3,4-Dichlorophenyl)-5-methyl-3-[2-(pyrrolidin-1-yl)ethoxy]-1 H-pyrazole
[4] 1-(3,4-Dichlorophenyl)-5-methyl-3-[3-(pyrrolidin-1-yl)propoxy]-1 H-pyrazole
[5] 1-{2-[1-(3,4-Dichlorophenyl)-5-methyl-1H-pyrazol-3-yloxy]ethyl}piperidine
[6] 1-{2-[1-(3,4-dichlorophenyl)-5-methyl-1H-pyrazol-3-yloxy]ethyl}-1H-imidazole
[7] 3-{1-[2-(1-(3,4-Dichlorophenyl)-5-methyl-1H-pyrazol-3-yloxy)ethyl]piperidin-4-yl}-3H-imidazo[4,5-b]pyridine
[8]1-{2-[1-(3,4-Dichlorophenyl)-5-methyl-1H-pyrazol-3-yloxy]ethyl]-4-methylpiperazine
[9] Ethyl 4-{2-[1-(3,4-dichlorophenyl)-5-methyl-1 H-pyrazol-3-yloxy]ethyl}piperazine carboxylate
[10] 1-(4-(2-(1-(3,4-dichlorophenyl)-5-methyl-1 H-pyrazol-3-yloxy)ethyl)piperazin-1-yl)ethanone
[11] 4-{2-[1-(4-Methoxyphenyl)-5-methyl-1 H-pyrazol-3-yloxy]ethyl}morpholine
[12] 1-(4-Methoxyphenyl)-5-methyl-3-[2-(pyrrolidin-1-yl)ethoxy]-1H-pyrazole
[13] 1-(4-Methoxyphenyl)-5-methyl-3-[3-(pyrrolidin-1-yl)propoxy]-1 H-pyrazole
[14] 1-[2-(1-(4-Methoxyphenyl)-5-methyl-1 H-pyrazol-3-yloxy)ethyl]piperidine
[15] 1-{2-[1-(4-Methoxyphenyl)-5-methyl-1 H-pyrazol-3-yloxy]ethyl}-1H-imidazole
[16] 4-{2-[1-(3,4-Dichlorophenyl)-5-phenyl-1H-pyrazol-3-yloxy]ethyl}morpholine
[17] 1-(3,4-Dichlorophenyl)-5-phenyl-3-[2-(pyrrolidin-1-yl)ethoxy]-1H-pyrazole
[18] 1-(3,4-Dichlorophenyl)-5-phenyl-3-[3-(pyrrolidin-1-yl)propoxy]-1 H-pyrazole
[19] 1-{2-[1-(3,4-Dichlorophenyl)-5-phenyl-1H-pyrazol-3-yloxy]ethyl}piperidine
[20] 1-{2-[1-(3,4-Dichlorophenyl)-5-phenyl-1 H-pyrazol-3-yloxy]ethyl}-1H-imidazole
[21]2-{2-[1-(3,4-dichlorophenyl)-5-phenyl-1H-pyrazol-3-yloxy]ethyl}-1,2,3,4-tetrahydroisoquinoline
[22] 4-{4-[1-(3,4-Dichlorophenyl)-5-methyl-1H-pyrazol-3-yloxy]butyl}morpholine
[23] 1-(3,4-Dichlorophenyl)-5-methyl-3-[4-(pyrrolidin-1-yl)butoxy]-1 H-pyrazole
[24] 1-{4-[1-(3,4-Dichlorophenyl)-5-methyl-1H-pyrazol-3-yloxy]butyl}piperidine
[25]1-{4-[1-(3,4-Dichlorophenyl)-5-methyl-1H-pyrazol-3-yloxy]butyl}-4-methylpiperazine
[26] 1-{4-[1-(3,4-Dichlorophenyl)-5-methyl-1H-pyrazol-3-yloxy]butyl}-1 H-imidazole
[27] 4-[1-(3,4-Dichlorophenyl)-5-methyl-1 H-pyrazol-3-yloxy]-N,N-diethylbutan-1-amine
[28]1-{4-[1-(3,4-dichlorophenyl)-5-methyl-1H-pyrazol-3-yloxy]butyl}-4-phenylpiperidine
[29] 1-{4-[1-(3,4-dichlorophenyl)-5-methyl-1H-pyrazol-3-yloxy]butyl}-6,7-dihydro-1 H-indol-4(5H)-one
[30] 2-{4-[1-(3,4-dichlorophenyl)-5-methyl-1H-pyrazol-3-yloxy]butyl}-1,2,3,4-tetrahydroisoquinoline
[31] 4-{2-[1-(3,4-dichlorophenyl)-5-isopropyl-1H-pyrazol-3-yloxy]ethyl}morpholine
[32]2-[1-(3,4-Dichlorophenyl)-5-isopropyl-1H-pyrazol-3-yloxy]-N,N-diethylethanamine
[33] 1-(3,4-Dichlorophenyl)-5-isopropyl-3-[2-(pyrrolidin-1-yl)ethoxy]-1H-pyrazole
[34] 1-(3,4-Dichlorophenyl)-5-isopropyl-3-[3-(pyrrolidin-1-yl)propoxy]-1 H-pyrazole
[35] 1-{2-[1-(3,4-Dichlorophenyl)-5-isopropyl-1H-pyrazol-3-yloxy]ethyl}piperidine
[36] 2-{2-[1-(3,4-dichlorophenyl)-5-isopropyl-1 H-pyrazol-3-yloxy]ethyl}-1,2,3,4-tetrahydroisoquinoline
[37] 4-{2-[1-(3,4-dichlorophenyl)-1H-pyrazol-3-yloxy]ethyl}morpholine
[38] 2-[1-(3,4-dichlorophenyl)-1 H-pyrazol-3-yloxy] N,N-diethylethanamine
[39] 1-(3,4-dichlorophenyl)-3-[2-(pyrrolidin-1-yl)ethoxy]-1H-pyrazole
[40] 1-{2-[1-(3,4-dichlorophenyl)-1H-pyrazol-3-yloxy]ethyl}piperidine
[41] 1-(3,4-dichlorophenyl)-3-[3-(pyrrolidin-1-yl)propoxy]-1 H-pyrazole
[42]1 -{2-[1-(3,4-Dichlorophenyl)-5-methyl-1H-pyrazol-3-yloxy]ethyl}piperazine
[43] 1-{2-[1-(3,4-Dichlorophenyl)-5-methyl-1H-pyrazol-3-yloxy]ethyl}pyrrolidin-3-amine
[44]4-{2-[1-(3,4-Dichlorophenyl)-4,5-dimethyl-1H-pyrazol-3-yloxy]ethyl}morpholine
[45]4-{2-[1-(3,4-Dichlorophenyl)-4,5-dimethyl-1H-pyrazol-3-yloxy]ethyl}morpholine
[46]2-[1-(3,4-Dichlorophenyl)-4,5-dimethyl-1H-pyrazol-3-yloxy]-N,N-diethylethanamine
[47] 1-(3,4-Dichlorophenyl)-4,5-dimethyl-3-[2-(pyrrolidin-1-yl)ethoxy]-1H-pyrazole
[48] 1-(3,4-Dichlorophenyl)-4,5-dimethyl-3-[3-(pyrrolidin-1-yl)propoxy]-1 H-pyrazole
[49] 1-{2-[1-(3,4-Dichlorophenyl)-4,5-dimethyl-1 H-pyrazol-3-yloxy]ethyl}piperidine
[50] 4-{4-[1-(3,4-dichlorophenyl)-1H-pyrazol-3-yloxy]butyl}morpholine
[51](2S,6R)-4-{4-[1-(3,4-dichlorophenyl)-1H-pyrazol-3-yloxy]butyl}-2,6-dimethylmorpholine
[52] 1-{4-[1-(3,4-Dichlorophenyl)-1H-pyrazol-3-yloxy]butyl}piperidine
[53] 1-(3,4-Dichlorophenyl)-3-[4-(pyrrolidin-1-yl)butoxy]-1 H-pyrazole
[55] 4-[1-(3,4-dichlorophenyl)-1 H-pyrazol-3-yloxy]-N,N-diethylbutan-1-amine
[56] N-benzyl-4-[1-(3,4-dichlorophenyl)-1H-pyrazol-3-yloxy]-N-methylbutan-1-amine
[57]4-[1-(3,4-dichlorophenyl)-1 H-pyrazol-3-yloxy]-N-(2-methoxyethyl)-N-methylbutan-1-amine
[58] 4-{4-[1-(3,4-dichlorophenyl)-1H-pyrazol-3-yloxy]butyl}thiomorpholine
[59]1-[1-(3,4-Dichlorophenyl)-5-methyl-3-(2-morpholinoethoxy)-1 H-pyrazol-4-yl]ethanone
[60]1-{1-(3,4-dichlorophenyl)-5-methyl-3-[2-(pyrrolidin-1-yl)ethoxy]-1H-pyrazol-4-yl}ethanone
[61] 1-{1-(3,4-dichlorophenyl)-5-methyl-3-[2-(piperidin-1-yl)ethoxy]-1H-pyrazol-4-yl}ethanone
[62] 1-{1-(3,4-dichlorophenyl)-3-[2-(diethylamino)ethoxy]-5-methyl-1H-pyrazol-4-yl}ethanone
[63] 4-{2-[5-Methyl-1-(naphthalen-2-yl)-1H-pyrazol-3-yloxy]ethyl}morpholine
[64] N,N-Diethyl-2-[5-methyl-1-(naphthalen-2-yl)-1H-pyrazol-3-yloxy]ethanamine
[65] 1-{2-[5-Methyl-1-(naphthalen-2-yl)-1H-pyrazol-3-yloxy]ethyl}piperidine
[66] 5-Methyl-1-(naphthalen-2-yl)-3-[2-(pyrrolidin-1-yl)ethoxy]-1H-pyrazole
or their pharmaceutically acceptable salts, estereoisomers, solvates or a prodrug thereof.

In a more preferred variant of the invention, the sigma ligand of formula (I) is 4-{2-[5-Methyl-1-(naphthalen-2-yl)-1H-pyrazol-3-yloxy]ethyl} morpholine. This particular compound is designated in the examples of the present invention as compound 63.

In a still more preferred variant of the invention, the sigma ligand of formula (I) is 4-{2-[5-Methyl-1-(naphthalen-2-yl)-1 H-pyrazol-3-yloxy]ethyl} morpholine hydrochloride.

Any compound referred to herein is intended to represent such specific compound as well as certain variations or forms. In particular, compounds referred to herein may have asymmetric centres and therefore exist in different enantiomeric or diastereomeric forms. Thus, any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same as, or different to, the stereoisomerism of the other double bonds of the molecule. Furthermore, compounds referred to herein may exist as atropisomers. All the stereoisomers including enantiomers, diastereoisomers, geometric isomers and atropisomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by 13C- or 14C-enriched carbon, or the replacement of at least one nitrogen by 15N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds of formula (I) and their salts or solvates can be prepared as disclosed in the previous application WO2006/021462.

In a preferred embodiment of the invention, the pain is induced by a chemotherapeutic drug selected from drugs derived from platinum, from plant alkaloids, thalidomide and derivatives, and terpenes (terpenoids).

More preferably the drugs derived from platinum are the commercially available cisplatin, carboplatin or oxaliplatin.

"Plant alkaloids" (and terpenoids) are alkaloids derived from plants that block cell division by preventing microtubule function. Since microtubules are vital for cell division, their inhibition also arrests cell mitosis. The main examples of plant alkaloids are vinca alkaloids and taxanes.

"Vinca alkaloids" bind to specific sites on tubulin, inhibiting the assembly of tubulin into microtubules (M phase of the cell cycle). They are derived from the Madagascar periwinkle, Catharanthus roseus (formerly known as Vinca rosea). The preferred vinca alkaloids include Vincristine, Vinblastine, Vinorelbine, and Vindesine.

"Taxanes" are derived from the Pacific yew tree, Taxus brevifolia. Taxanes enhance the stability of microtubules, preventing the separation of chromosomes during anaphase. Preferred taxanes in this invention include Paclitaxel and Docetaxel.

Examples of chemotherapeutic drugs (by their trademarks) that can induce pain that can be prevented or treated with the sigma ligands of formula (I) are:
13-cis-Retinoic Acid, 2-CdA, 2-Chlorodeoxyadenosine, 5-fluorouracil 5-FU, 6-Mercaptopurine, 6-MP, 6-TG 6-Thioguanine, Abraxane, Accutane ®, Actinomycin-D, Adriamycin ®, Adrucil ®, Agrylin ®, Ala-Cort ®, Aldesleukin, Alemtuzumab, ALIMTA, Alitretinoin, Alkaban-AQ ®, Alkeran ®, All-transretinoic acid, Alpha interferon, Altretamine, Amethopterin, Amifostine, Aminoglutethimide, Anagrelide, Anandron ®, Anastrozole, Arabinosylcytosine, Ara-C, Aranesp ®, Aredia ®, Arimidex ®, Aromasin ®, Arranon ®, Arsenic trioxide, Asparaginase, ATRA, Avastin ®, Azacitidine"BCG, BCNU, Bevacizumab, Bexarotene, BEXXAR ®, Bicalutamide, BiCNU, Blenoxane ®, Bleomycin, Bortezomib, Busulfan, Busulfex ®"C225, Calcium Leucovorin, Campath ®, Camptosar®, Camptothecin-11, Capecitabine, Carac TM, Carboplatin, Carmustine, Carmustine wafer, Casodex®, CC-5013, CCNU (o), CDDP (t), CeeNU (t), Cerubidine (t), cetuximab, Chlorambucil, Cisplatin, Citrovorum Factor, Cladribine, Cortisone, Cosmegen (t), CPT-11 (o), Cyclophosphamide, Cytadren (t), Cytarabine, Cytarabine liposomal, Cytosar-U (t), Cytoxan ®, Dacarbazine, Dactinomycin, Darbepoetin alfa, Daunomycin, Daunorubicin, Daunorubicin hydrochloride (t), Daunorubicin liposomal, DaunoXome (t), Decadron, Delta-Cortef (t), Deltasone (t), Denileukin, diftitox, DepoCyt (t), Dexamethasone, Dexamethasone acetate, dexamethasone sodium phosphate, Dexasone (t), Dexrazoxane, DHAD (o), DIC (t), Diodex (t), Docetaxel, Doxil (t), Doxorubicin, Doxorubicin liposomal, Droxia (t), DTIC, DTIC-Dome (t), Duralone (t), Efudex (t), Eligard (t), Ellence (t), Eloxatin (t), Elspar (t), Emcyt (t), Epirubicin, Epoetin alfa, Erbitux, Erlotinib, Erwinia L-asparaginase (t), Estramustine, Ethyol, Etopophos (t), Etoposide, Etoposide phosphate (t), Eulexin (t), Evista (t), Exemestane, Fareston (t), Faslodex (t), Femara ®, Filgrastim, Floxuridine, Fludara (t), Fludarabine, Fluoroplex (t), Fluorouracil, Fluorouracil (cream), Fluoxymesterone, Flutamide, Folinic Acid (o), FUDR (t), Fulvestrant, G-CSF (t), Gefitinib, Gemcitabine, Gemtuzumab ozogamicin, Gemzar (t), GleevecTM, Gliadel wafer (t), GM-CSF (o), Goserelin, granulocyte - colony stimulating factor (t), Granulocyte macrophage colony stimulating factor (o), Halotestin (t), Herceptin (t), Hexadrol (t), Hexalen (t), Hexamethylmelamine (t), HMM (t), Hycamtin (t), Hydrea (t), Hydrocort Acetate (t), Hydrocortisone, Hydrocortisone sodium phosphate, Hydrocortisone sodium succinate, Hydrocortone phosphate (t), Hydroxyurea, Ibritumomab, Ibritumomab Tiuxetan, Idamycin ®, Idarubicin Ifex®, IFN-alpha, Ifosfamide, IL-11, IL-2, Imatinib mesylate, Imidazole Carboxamide, Interferon alfa, Interferon Alfa-2b (PEG conjugate) (o), Interleukin - 2 (t), Interleukin-11 (o), Intron A® (interferon alfa-2b), Iressa ®, Irinotecan, Isotretinoin, Kidrolase (t), Lanacort (t), L-asparaginase (t), LCR (o), Lenalidomide (Lenolidamide), Letrozole, Leucovorin, Leukeran (t), Leukine (t), Leuprolide, Leurocristine (o), Leustatin (t), Liposomal Ara-C (t), Liquid Pred (t), Lomustine, L-PAM (o), L-Sarcolysin (o), Lupron (t), Lupron Depot (t), Matulane (t), Maxidex (t), Mechlorethamine, Mechlorethamine Hydrochloride, Medralone (t), Medrol ®, Megace (t), Megestrol, Megestrol Acetate (o), Melphalan, Mercaptopurine, Mesna, Mesnex (t), Methotrexate, Methotrexate Sodium (o), Methylprednisolone, Meticorten (t), Mitomycin, Mitomycin-C (o), Mitoxantrone, M-Prednisol (t), MTC (o), MTX (o), Mustargen (t), Mustine, Mutamycin (t), Myleran (t), Mylocel (t), Mylotarg (t), Navelbine (t), Nelarabine, Neosar (t), Neulasta (t), Neumega (t), Neupogen (t), Nexavar®, Nilandron (t), Nilutamide, Nipent®, Nitrogen Mustard (o), Novaldex (t), Novantrone (t), Octreotide, Octreotide acetate (o), Oncospar (t), Oncovin (t), Ontak (t), Onxal (t), Oprevelkin, Orapred (t), Orasone (t), Oxaliplatin, Paclitaxel, Paclitaxel Protein-bound, Pamidronate, Panretin (t), Paraplatin (t), Pediapred (t), PEG Interferon, Pegaspargase, Pegfilgrastim, PEG-INTRON (t), PEG-L-asparaginase, Pemetrexed, Pentostatin, Phenylalanine Mustard (o), Platinol (t), Platinol-AQ (t), Prednisolone, Prednisone, Prelone (t), Procarbazine, PROCRIT ®, Proleukin (t), Prolifeprospan 20 with Carmustine implant (t), Purinethol (t), Raloxifene, Revlimid ®, Rheumatrex (t), Rituxan (t), Rituximab, Roferon-A®, (interferon alfa-2a) Rubex (t), Rubidomycin hydrochloride (t), Sandostatin ®, Sandostatin LAR (t), Sargramostim, Solu-Cortef (t), Solu-Medrol (t), Sorafenib, STI-571, Streptozocin, SU11248, Sunitinib, Sutent ®, Tamoxifen, Tarceva ®, Targretin (t), Taxol ®, Taxotere (t), Temodar®, Temozolomide, Teniposide, TESPA (o), Thalidomide, Thalomid ®, TheraCys (t), Thioguanine, Thioguanine Tabloid (t), Thiophosphoamide (o), Thioplex (t), Thiotepa, TICE ®, Toposar (t), Topotecan, Toremifene, Tositumomab, Trastuzumab, Tretinoin, Trexall (t), Trisenox (t), TSPA (o), VCR (o), Velban (t), Velcade ®, VePesid (t), Vesanoid (t), Viadur (t), Vidaza (t), Vinblastine, Vinblastine Sulfate (o), Vincasar Pfs (t), Vincristine, Vinorelbine, Vinorelbine tartrate (o), VLB (o), VM-26 (o), VP-16 (t), Vumon (t), Xeloda ®, Xyotax, Zanosar (t), Zevalin TM, Zinecard (t), Zoladex ®, Zoledronic acid, and Zometa ®.

Another drugs used in cancer-therapy (mostly as chemotherapeutics) are:
(as trademarks): Aldara, Alimta, Androcur, Arimidex, Borea, Caelyx, Campto, Casodex, Decapeptyl, Eloxatin, Eutirox, Faslodex, Femara, Gemzar, Gonapeptyl, Grisetin, Herceptin, Isovorin, Lysodren, Megefren, Metvix, Navelbine, Novaldex, Novantrone, Paraplatin, Procrin, Prostacur, Suprefact, Tamoxifeno Funk, Taxol, Taxotere, Testex, Elmu/Prolongatum, Tomudex, Utefos, Vepesid, Xeloda, Zoladex;
(as active compounds): Anastrozole, Bicalutamide, Busereline, Capecetabine, Cisplatin, Carboplatin, Desoxorubicin, Docetaxel, Etoposid, Fulvestrant, Gemcitabine, Gosereline, Irinotecan, Letrozole, Leuproreline, Megestrol, Mitotane, Mitoxantrone, Oxaliplatin, Paclitaxel, Pemetrexed, Raltitrexed, Tamoxiphen, Tegafur, Triptoreline, Vincristine, Vinblastine, Vinorelbine, and Vindesine.

According to the invention, the chemotherapeutic drug is selected from taxanes, vinca alkaloids, drugs derived from platinum or thalidomide and its derivatives. Preferably, the chemotherapeutic drug is selected from paclitaxel, oxaliplatin, cisplatin, vincristine, bortezomib, thalidomide or lenolidamide,

In a more preferred embodiment of the invention, the chemotherapeutic drug is a taxane, particularly Paclitaxel. Paclitaxel (Taxol®) is one of the most effective and commonly used antineoplasic drugs for the treatment of solid tumours. It has two serious side effects, myelosupression and peripheral neurotoxicity. The granulocyte colony-stimulating factor effectively counteracts the neutropenia in most patients. However, there are no acceptable therapies to prevent or minimize the nerve damage, making neurotoxicity a significant dose-limiting side effect (Rowinsky et al., Semin. Oncol., 1993a 29 4 Suppl. 3, 1-15; Rowinsky et al., J. Clin. Oncol., 1993b, 11(10), 2010-20; Wasserheit et al., J. Clin. Oncol., 1996, 14(7), 1993-9; Gordon et al., J. Clin. Oncol., 1997, 15(5), 1965-73; Mielke et al., Eur. J. Cancer, 2006, 42(1), 24-30). Paclitaxel-induced neurotoxicity typically presents as a sensory neuropathy, with the most common complaints being numbness, tingling, burning pain and cold allodynia (Rowinsky et al., Semin. Oncol., 1993a 29 4 Suppl. 3, 1-15; Chaudhry et al., Ann. Neurol., 1994, 35(3), 304-11; Forsyth et al., J. Neurooncol., 1997, 35(1), 47-53; Dougherty et al., Pain, 2004, 109(1-2), 132-42). Sensory symptoms usually start symmetrically in the feet, but sometimes appear simultaneously in both hands and feet (Rowinsky et al., Semin. Oncol., 1993a 29 4 Suppl. 3, 1-15; Quasthoff and Hartung, J. Neurol., 2002, 249(1), 9-17; Mielke et al., Eur. J. Cancer, 2006, 42(1), 24-30). A clinically significant number of patients with paclitaxel-induced neuropathy experience neuropathic pain. For example, in a study of 27 patients treated with paclitaxel doses of 135, 175 and 250-300 mg/m², neuropathic symptoms occurred in 50, 79 and 100% of patients, progressing to dose-limiting neurotoxicity in 0, 21 and 71% of patients, respectively (Postma et al., Ann. Oncol., 1995, 6(5), 489-94).

In another more preferred embodiment of the invention, the chemotherapeutic drug is a platinum-containing drug, particularly Oxaliplatin or Cisplatin. These platinum-based chemotherapies are a mainstay for the treatment of solid tumors, especially colorectal, but also ovarian, testicular, bladder and lung cancer, but their clinical use is severely curtailed by dose-limiting neurotoxicity. The neurotoxicity induced by this class of anticancer drugs is characterized by a dose-dependent painful peripheral sensory neuropathy presenting with symptoms of neuropathic pain (i.e., numbness, tingling, burning pain, allodynia and hyperalgesia) in the distal extremities. Oxaliplatin treatment results in two forms of neurotoxicity: acute and chronic. The acute form occurs in >90% of patients and may begin during the infusion or within hours of completion, is usually self-limited, and may be exacerbated by exposure to cold. Chronic neuropathy is cumulative and is most commonly seen in patients who have received total doses ≥540 mg/m². Although it is a sensory neuropathy, the intensity can increase to the point that it impairs physical functions, such as holding objects and writing (Cersosimo RJ., Ann. Pharmacother., 2005, 39(1), 128-135).

Preferred combinations of the invention comprise the combination of 4-{2-[5-Methyl-1-(naphthalen-2-yl)-1H-pyrazol-3-yloxy]ethyl}morpholine (compound 63) with a chemotherapeutic drug selected from Paclitaxel, Oxaliplatin, Cisplatin and Vincristine.

More preferred combinations of the invention comprise the combination of 4-{2-[5-Methyl-1-(naphthalen-2-yl)-1H-pyrazol-3-yloxy]ethyl}morpholine with Paclitaxel, the combination of 4-{2-[5-Methyl-1-(naphthalen-2-yl)-1H-pyrazol-3-yloxy]ethyl}morpholine with Oxaliplatin and the combination of 4-{2-[5-Methyl-1-(naphthalen-2-yl)-1H-pyrazol-3-yloxy]ethyl}morpholine with Cisplatin.

In additional preferred embodiments, the preferences described above for the different groups and substituents in the formulae above as well as for the chemotherapeutic drug are combined. The present invention is also directed to such combinations.

The combination of the invention may be formulated for its simultaneous, separate or sequential administration, with at least a pharmaceutically acceptable carrier, additive, adjuvant or vehicle. This has the implication that the combination of the two active compounds may be administered:
- as a combination that is being part of the same medicament formulation, the two active compounds being then administered always simultaneously.
- as a combination of two units, each with one of the active substances giving rise to the possibility of simultaneous, sequential or separate administration.

In a particular embodiment, the sigma ligand of formula (I) is independently administered from the chemotherapeutic drug (i.e in two units) but at the same time.

In another particular embodiment, the sigma ligand of formula (I) is administered first, and then the chemotherapeutic drug is separately or sequentially administered.

These particular ways of administration are preferably used to prevent the pain developing as a consequence of chemotherapy.

In yet another particular embodiment, the chemotherapeutic drug is administered first, and then the sigma ligand of formula (I) is administered, separately or sequentially, as defined.

This particular way of administration is preferably used to treat the pain developing as a consequence of chemotherapy.

The auxiliary materials or additives can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical combination in accordance with the invention can be adapted to any form of administration, be it orally or parenterally, for example pulmonar, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application.

Suitable preparations for oral applications are tablets, pills, chewing gums, capsules, granules, drops or syrups.

Suitable preparations for parenteral applications are solutions, suspensions, reconstitutable dry preparations or sprays.

The combination of the invention may be formulated as deposits in dissolved form or in patches, for percutaneous application.

Skin applications include ointments, gels, creams, lotions, suspensions or emulsions.

Suitable form of rectal application is by means of suppositories.

As mentioned above, the compound of formula (I) is suited for its use in the prevention or treatment of pain induced by chemotherapy. The pain is induced by a chemotherapeutic drug selected from taxanes, vinca alkaloids, drugs derived from platinum or thalidomide and its derivatives. Preferably, the chemotherapeutic drug is selected from paclitaxel, oxaliplatin, cisplatin, vincristine, bortezomib, thalidomide or lenolidamide, more preferably it is selected from paclitaxel. oxaliplatin or cisplatin.

In addition, the combination of at least one compound of general formula (I) and at least one chemotherapeutic drug is suited for its use in the prevention or treatment of pain developing as a consequence of chemotherapy. This combination for its use in the prevention or treatment of pain can be administered simultaneously, separately or sequentially. Single or repeated administrations are considered for the prevention or treatment of pain developing as a consequence of chemotherapy.

Therefore, another aspect of the invention refers to a combination as defined above for its use in the prevention or treatment of pain induced by chemotherapy.

In a particular embodiment, said combination is used in the prevention of pain induced by chemotherapy.

In another particular embodiment, said combination is used in the treatment of pain induced by chemotherapy.

In a particular embodiment of the present invention, the pain is peripheral neuropathic pain, allodynia, causalgia, hyperalgesia, hyperesthesia, hyperpathia, neuralgia, neuritis or neuropathy. More preferably, the pain is cold-allodynia or mechanical allodynia.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention this term is to be treated as synonymous to "Neurogenic Pain" which is defined by the IASP as "pain initiated or caused by a primary lesion, dysfunction or transitory perturbation in the peripheral or central nervous system". Neuropathic pain according to this invention is restricted to the neuropathic pain resulting from chemotherapy, meaning caused by the use of a chemotherapeutic drug in chemotherapy. The most likely cause of this pain is the chemotherapeutic drug neurotoxicity, and more specifically, its peripheral neurotoxicity.

According to the IASP "allodynia" is defined as "a pain due to a stimulus which does not normally provoke pain" (IASP, Classification of chronic pain, 2nd Edition, IASP Press (2002), 210). According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperalgesia" is defined as "an increased response to a stimulus which is normally painful" (IASP, Classification of chronic pain, 2nd Edition, IASP Press (2002), 211).

According to the IASP "hyperesthesia" is defined as "increased sensitivity to stimulation, excluding the senses" (IASP, Classification of chronic pain, 2nd Edition, IASP Press (2002), 211).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold Increased response | Stimulus and response rate may be the same or different |

According to the IASP "neuralgia" is defined as "pain in the distribution of a nerve or nerves" (IASP, Classification of chronic pain, 2nd Edition, IASP Press (2002), 212).

According to the IASP "neuritis" is defined as "inflammation of a nerve or nerves" (IASP, Classification of chronic pain, 2nd Edition, IASP Press (2002), 212).

According to the IASP "neuropathy/neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

In one embodiment of the invention It is preferred that the compound of formula (I) is used in therapeutically effective amounts. The physician will determine the dosage of the present therapeutic agents which will be most suitable and it will vary with the form of administration and the particular compound chosen, and furthermore, it will vary with the patient under treatment, the age of the patient, the type of cancer and pain being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

For example, the dosage regime that must be administered to the patient will depend on the patient's weight, the type of application, the condition and severity of the disease. A preferred dosage regime of comprises an administration of a compound of formula (I) within a range of 0.5 to 100 mg/kg and of the chemotherapeutic drug from 0.15 to 15 mg/kg and it is administered daily in one or several doses.

The following examples and figures are merely illustrative of certain embodiments of the invention and cannot be considered as restricting it in any way.

### Examples

### Example 1. Synthesis of 4-{2-[5-Methyl-1-(naphthalen-2-yl)-1H-pyrazol-3-yloxy]ethyl} morpholine (compound 63) and its hydrochloride salt

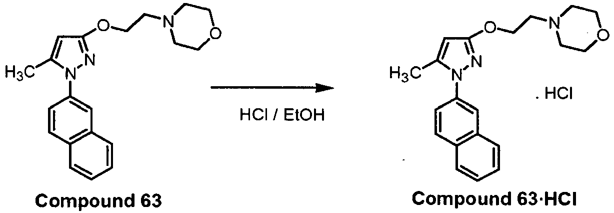

Compound 63 can be can be prepared as disclosed in the previous application WO2006/021462. Its hydrochloride can be obtained according the following procedure:
Compound 63 (6,39 g) was dissolved in ethanol saturated with HCl, the mixture was stirred then for some minutes and evaporated to dryness. The residue was crystallized from isopropanol. The mother liquors from the first crystallization afforded a second crystallization by concentrating. Both crystallizations taken together yielded 5,24 g (63 %) of the corresponding hydrochloride salt (m.p. = 197-199°C.)
¹H-NMR (DMSO-d₆) δ ppm: 10,85 (bs, 1 H), 7,95 (m, 4H), 7,7 (dd, J=2,2, 8,8 Hz, 1 H), 7,55 (m, 2H), 5,9 (s, 1 H), 4,55 (m, 2H), 3,95 (m, 2H), 3,75 (m, 2H), 3,55-3,4 (m, 4H), 3,2 (m, 2H), 2,35 (s, 3H).
HPLC purity: 99.8%

### Example 2. Assessment of the preventive and curative antiallodynic effect of compound 63 in a model of paclitaxel-induced neuropathy in mice.

### Common procedures

Experiments were performed in CD-1 mice (Charles River, U.S.A.) with at least n = 10/experimental group. Paclitaxel-induced painful peripheral neuropathy was produced by i.p. administration of paclitaxel once daily during 5 days. Control animals received the same volume of solvent (a mixture of ethanol and cremophor EL)

Mechanical allodynia was evaluated with an electronically driven Von Frey filament (Dynamic Plantar Aesthesiometer, Ugo Basile, Varese, Italy) as previously described (Nieto et al., Pain, 2008, 137(3), 520-31), and cold allodynia was evaluated using the acetone drop method (Nieto et al., Pain, 2008, 137(3), 520-31). The sigma receptor ligand compound 63 (4-{2-[5-Methyl-1-(naphthalen-2-yl)-1H-pyrazol-3-yloxy]ethyl} morpholine) was injected s.c. either 30 min before each paclitaxel injection to test whether the sigma receptor ligand affects the development of the painful peripheral neuropathy or at day 10 (when paclitaxel injections have ended and the neuropathy is fully developed). In addition, in order to study the influence of the sigma 1 receptor in this process the difference in development of allodynia was determined using wild-type mice and sigma 1-receptor knock-out-mice.

Mice weighing 25-30 g were used. The animals were housed in colony cages with free access to food and water prior to the experiments. They were maintained in temperature- and light-controlled rooms (22 ± 1°C, lights on at 08.00 h and off at 20.00 h, air replacement every 20 min). Testing took place during the light phase (from 9.00 h to 15.00 h).

Paclitaxel was dissolved in a solution made up of 50% Cremophor EL and 50% absolute ethanol to obtain a concentration of 6 mg/ml. This paclitaxel solution was conserved at -20 °C during a maximum of 14 days and was diluted in normal saline (NaCl 0.9%), just before administration, to a final concentration of 2 mg/10 ml. The vehicle of paclitaxel was diluted at the time of injection with saline (NaCl 0.9%) in the same proportion as the paclitaxel solution.

Paclitaxel (2 mg/kg) was administered intraperitoneously (i.p.), in a volume of 10 ml/kg, once per day for five consecutive days. Therefore, the cumulative dose was 10 mg/kg per mouse. In the control group the vehicle of paclitaxel was administered following the same schedule. The same schedules of paclitaxel injection also applied when testing groups of sigma-1-knock-out mice against groups of wild-type mice.

Compound 63 was dissolved in normal saline just before administration and applied at doses of 8, 16, 32 or 64 mg/kg subcutaneously.

The effects of Compound 63 on paclitaxel-induced neuropathic pain were examined in two different ways. To evaluate the effect of the sigma receptor antagonist Compound 63 on the development of paclitaxel-induced pain, the animals received an s.c. injection of compound 63 30 min before each i.p. injection of paclitaxel for five consecutive days. The response of the animals to the different nociceptive stimuli was tested subsequently during 2-4 weeks, depending on the test (see below), without any additional treatment. Each animal was tested only in one nociceptive model. To test the effect of compound 63 on the *expression* of paclitaxel-induced pain, a single compound 63 injection was performed on day 10, a day of maximum expression of mechanical allodynia or cold allodynia (see figures for details). Each animal received only one dose of compound 63 and was tested in only one nociceptive model.

**Procedure for assessment of cold allodynia.** Cold allodynia was tested as previously described by Nieto et al., Pain, 2008, 137(3), 520-31, by gently touching the plantar skin of the hind paws with an acetone bubble formed with a syringe connected to a thin polyethylene tube. The mice were housed and habituated for 30 min in transparent plastic boxes (7 x 7 x 13 cm) with a floor made of wire mesh. After the adaptation period, acetone was applied alternately three times to each paw at intervals of 30 s, and the duration of licking or biting were recorded. A small mirror was placed behind the chambers to allow clear observation of the paws. The time spent licking or biting the paw was recorded by a stopwatch and represented as the cumulative time of licking/biting in the six measurements. Since licking persisting more than 10 s in the experiments was very unusual, a cut-off time of 10 s was used for each trial.

To elucidate the time-course of paclitaxel-induced cold allodynia in control mice, the animals were tested previously to the paclitaxel administration (pretreatment value, 3 days before first paclitaxel-treatment) and on different days (days 7, 10, 14, 17, 21 and 24) after the first paclitaxel or vehicle injection.

The same procedure was followed to compare sigma-1-knock-out mice to wild type mice, thus elucidating the time-course of paclitaxel-induced cold allodynia in control mice. Accordingly, the animals (two equal groups of knock-out and wild-type mice were tested previously to paclitaxel administration (pretreatment value, 3 days before first paclitaxel-treatment) and on different days (days 7, 10, 14, 17, 21 and 24) after the first paclitaxel or vehicle injection.

The same procedure was followed to test the effect of compound 63 on the development of cold allodynia, but in this case, compound 63 or its vehicle was injected s.c. 30 min before each of the 5 paclitaxel i.p. injections. Once more the animals were tested previously to the paclitaxel/ compound 63 administration (pretreatment value, 3 days before first paclitaxel/ compound 63 treatment) and on different days (days 7, 10, 14, 17, 21 and 24) after the first paclitaxel/ compound 63 or vehicle injection. The effect of compound 63 on the expression of paclitaxel-induced cold allodynia was evaluated on day 10, because the maximum allodynic effect was observed on that day. Therefore, the day 10, after the habituation period to the apparatus, baseline latencies were recorded, 30 min later compound 63 or saline was injected s.c. and paw withdrawal latencies were assessed again 60, 120 and 180 minutes after the injection. Around 33 % of the control animals treated with paclitaxel did not show cold allodynia; therefore, it was differentiated between 'responders' and 'non-responders' mice in this test. The 'non-responders' mice were easily identified because they spent less than 2 s licking/biting the paw stimulated with acetone on days 7 and 10 after paclitaxel administration. The 'non-responder' animals were not used to test the effect of compound 63 on the expression of cold allodynia since they do not express enough cold allodynia.

**Procedure for assessment of mechanical allodynia.** To assess mechanical allodynia, paw withdrawal thresholds were measured using a Dynamic Plantar Aesthesiometer (Ugo Basile, Italy). The electronic Von Frey device employs a single nonflexible filament which applies a progressively increasing force (from 0 to 10 g) against the plantar surface of the hind paw over a 20 s period. The nocifensive withdrawal reflex automatically turns off the stimulus and the mechanical threshold value is showed in a screen. The day of the experiment, mice were placed individually in test compartments (9 x 9 x 14 cm) with a wire mesh bottom and allowed to acclimatize to them for 2 h. After habituation, each mouse was tested three times alternately in each hind paw.

To elucidate the time-course of paclitaxel-induced mechanical allodynia in control mice, the animals were tested previously to the paclitaxel administration (pretreatment value; day 3 before paclitaxel-treatment) and on different days (days 7, 10, 14 and 17) after the first paclitaxel or vehicle injection.

The same procedure was followed to compare sigma-1-knock-out mice to wild type mice, thus elucidating the time-course of paclitaxel-induced mechanical allodynia in control mice. Accordingly, the animals (two equal groups of knock-out and wild-type mice were tested previously to the paclitaxel administration (pretreatment value, 3 days before first paclitaxel-treatment) and on different days (days 7, 10, 14 and 17) after the first paclitaxel or vehicle injection.

The same procedure was followed to test the effect of compound 63 on the development of paclitaxel-induced mechanical allodynia. In this case, compound 63 or its vehicle was injected s.c. 30 min before each of the 5 paclitaxel i.p. injections. Once more the animals were tested previously to the paclitaxel/ compound 63 administration (pretreatment value, 3 days before first paclitaxel/ compound 63 treatment) and on different days (days 7, 10, 14 and 17) after the first paclitaxel/ compound 63 or vehicle injection. The effect of compound 63 on the expression of paclitaxel-induced mechanical allodynia was evaluated on day 10, because the maximum change of the mechanical threshold was observed on that day. Therefore, the day 10, after the habituation period to the apparatus, baseline latencies were recorded, 30 min later compound 63 or saline was injected s.c. and paw withdrawal latencies were assessed again 30, 60, 90, 120 and 180 minutes after the injection. Most animals (96 %) treated with paclitaxel showed a reduction of the mechanical threshold; those animals that did not show mechanical allodynia were not used to test the effect of compound 63 on the expression of paclitaxel-induced mechanical allodynia.

### Results on cold-allodynia

### A) Time course of cold allodynia induced by paclitaxel in control mice.

Animals were treated once daily from days 1 to 5 with paclitaxel (2 mg/kg) or its vehicle via i.p. The duration of hind paw licking/biting in the acetone test was recorded 3 days before (PRE) and at several days after the first injection of paclitaxel or its vehicle. Each animal was tested only in one nociceptive model. The values obtained on the pre-treatment day in paclitaxel- and vehicle-treated animals were not significantly different in the acetone test. Administration during 5 days of paclitaxel-vehicle did not significantly modify the response of the animals in the acetone test at any post-treatment day in comparison to the pre-treatment value.

In the acetone test (Fig. 1), administration of paclitaxel (2 mg/kg, i.p.) once daily during 5 days allowed to distinguish between two groups of animals depending on their response. Most animals (67%) treated with paclitaxel increased significantly (p < 0.01) the time spent licking/biting the paw stimulated (Fig. 1) and the frequency of paw licking/biting at all the post-treatment days, in comparison to the pre-treatment day value. These animals constitute the paclitaxel-responder animals. On the other hand, a 33% of paclitaxel-treated animals did not show cold allodynia, and their response to acetone was indistinguishable from that of animals treated with the paclitaxel-vehicle (Fig. 1). When the duration of licking/biting values among the different groups obtained on the same day of evaluation were compared, statistically significant differences between paclitaxel-responder and the other two groups (paclitaxel-non-responder or paclitaxel-vehicle) were observed for each day of evaluation after treatment (Fig. 1). Paclitaxel-induced cold allodynia was maximal 10-14 days after the first injection of the antineoplastic (Fig. 1); therefore, the effect of compound 63 on the expression of cold allodynia was evaluated on day 10.

### B) Effect of compound 63 on the expression of paclitaxel-induced cold allodynia.

Animals were treated once daily from days 1 to 5 with paclitaxel i.p., and on day 10 received a single s.c. injection of Compound 63 or saline. The duration of hind paw licking/biting was recorded in each animal 3 days before (PRE) and 10 days after the first injection of paclitaxel. On that day, duration of hind paw licking/biting was recorded immediately before (time 0) and at several times (60, 120 and 180 min) after the injection of compound 63 or saline.

The duration of paw licking/biting on day 10, before the treatment with the tested drug or saline, were significantly different from their values on pre-treatment day in all groups of animals treated. As expected, paclitaxel induced a cold allodynia 10 days after its first injection. A single s.c. injection of saline on day 10 did not significantly modify the expression of paclitaxel-induced cold allodynia. The acute treatment with various amounts of compound 63 (32-128 mg/kg) inhibited the expression of paclitaxel-induced cold allodynia. This effect of compound 63 was dose-dependently, significantly different from that of saline (Fig. 2).

### C) Effect of compound 63 on the response to acetone of paclitaxel-vehicle treated mice.

Animals were treated once daily from days 1 to 5 with paclitaxel-vehicle i.p., and on day 10 received a single s.c. injection of compound 63 or saline. The duration of hind paw licking/biting was recorded in each animal 3 days before (PRE) and 10 days after the first injection of paclitaxel-vehicle. On that day, duration of hind paw licking/biting was recorded immediately before (time 0) and at several times (60, 120 and 180 min) after the injection of compound 63 or saline. As expected, the animals treated with paclitaxel-vehicle did not develop cold allodynia (i.e. the duration of acetone-induced paw licking/biting did not increase) when evaluated 10 days after the first dose of paclitaxel-vehicle was administered (Fig. 3).

An acute treatment with compound 63 (64 mg/kg, s.c.) did not affect the response to acetone of control (paclitaxel-vehicle treated) animals (Fig. 3).

### D) Effect of compound 63 on the development of paclitaxel-induced cold allodynia.

Mice were treated once daily from days 1 to 5 with an s.c. injection of compound 63 (64 mg/kg) or saline 30 min before each i.p. injection of paclitaxel (2 mg/kg). The response was recorded in each animal 3 days before (PRE) and on different days after the first injection of paclitaxel + compound 63 or paclitaxel + saline. The pre-treatment values were similar in all the experimental groups in the acetone test (Fig. 4).

As expected, the group of animals treated s.c. with saline 30 min before each dose of paclitaxel developed cold allodynia (manifested by an increase in the duration of acetone-induced paw licking/biting) when evaluated during 24 days after the first dose of paclitaxel was administered (Fig. 4). Further, the maximum cold allodynia was found on days 10-14 after the first dose of the antineoplastic (Fig. 4).

Co-administration of paclitaxel (2 mg/kg, i.p.) and the σ₁ receptor antagonist compound 63 (64 mg/kg, s.c.) inhibited the development of cold allodynia induced by paclitaxel (Fig. 4).

### E) Effect of co-administration of paclitaxel-vehicle and compound 63 on the response to acetone.

Mice were treated once daily from days 1 to 5 with an s.c. injection of compound 63 (64 mg/kg) or saline 30 min before each i.p. injection of paclitaxel vehicle. The response was recorded in each animal 3 days before (PRE) and on different days after the first injection of paclitaxel vehicle + compound 63 or paclitaxel vehicle + saline. As expected, none the groups of animals treated with paclitaxel-vehicle developed cold allodynia (i.e. they did not show an increase in the duration of acetone-induced paw licking/biting) when evaluated during 24 days after the first dose of paclitaxel-vehicle . was administered.

Co-administration of paclitaxel-vehicle and compound 63 (64 mg/kg, s.c.) did not significantly modify the response of the animals to acetone in comparison to that found in animals co-administered with paclitaxel-vehicle and saline (Fig. 5).

### F) Effect of paclitaxel and paclitaxel-vehicle in wild type and sigma-1 receptor knockout mice.

Animals were treated once daily from days 1 to 5 with paclitaxel (2 mg/kg) i.p. The duration of hind paw licking or biting in the acetone test were recorded 3 days before (PRE) and on several days after the first injection of paclitaxel. The duration of acetone-induced paw licking/biting before (PRE) the treatment with paclitaxel was very similar in wild type and sigma-1 knockout mice (Fig. 6).

The response to acetone (duration of hind paw licking/biting) was increased in wild-type mice from days 7 to 21 after the first dose of paclitaxel (Fig. 6). In contrast, the duration of acetone-induced licking/biting was not modified by paclitaxel treatment in σ₁ receptor knockout. Consequently, σ₁ receptor knockout mice did not express and/or did not develop paclitaxel-induced cold allodynia.

On the other hand, the animals treated with paclitaxel-vehicle did not develop cold allodynia (Fig. 7) and the response to acetone of paclitaxel-vehicle-treated animals was the same in wild type and σ₁ receptor knockout mice (Fig. 7).

### Results on mechanical allodynia

### G) Time course of mechanical allodynia induced by paclitaxel in control mice.

Animals were treated once daily from days 1 to 5 with paclitaxel (2 mg/kg) or its vehicle i.p. The threshold force for hind paw withdrawal in the Dynamic Plantar Aesthesiometer were recorded 3 days before (PRE) and on several days after the first injection of paclitaxel or its vehicle. Administration of paclitaxel (2 mg/kg, i.p., during 5 days) induced mechanical allodynia in mice, since it significantly reduced the threshold force for paw withdrawal in the Von Frey test on day 10, in comparison with both the pre-treatment day value and the value obtained the same day in the paclitaxel-vehicle treated animals (Fig. 8). Therefore, the effect of compound 63 on the expression of mechanical allodynia was tested on day 10. In contrast to cold allodynia results, only about 4% of mice treated with paclitaxel were considered non-responder animals; i.e. their mechanical threshold values on day 10 were not lower than their pretreatment values (obtained 3 days before paclitaxel treatment was started).

### H) Effect of compound 63 on the expression of paclitaxel-induced mechanical allodynia.

Animals were treated once daily from days 1 to 5 with paclitaxel and on day 10 received a single s.c. injection of compound 63 or saline. The threshold force for hind paw withdrawal was recorded in each animal 3 days before (PRE) and 10 days after the first injection of paclitaxel. On that day, paw withdrawal latency was recorded immediately before (time 0) and at several times (30, 60, 90, 120 and 180 min) after the injection of compound 63 or saline. The threshold forces of paw withdrawal on day 10, before the treatment with the tested drug or saline, were significantly different from their values on pre-treatment day in all groups of animals treated. Therefore, paclitaxel induced mechanical allodynia 10 days after its first injection. A single s.c. injection of saline on day 10 did not significantly modify the expression of paclitaxel-induced mechanical allodynia (Fig. 9).

An acute treatment with compound 63 (64 mg/kg, s.c.) inhibited the expression of mechanical allodynia induced by paclitaxel (Fig. 9).

### I) Effect of compound 63 on the threshold force for hind paw withdrawal of paclitaxel-vehicle treated mice.

Animals were treated once daily from days 1 to 5 with paclitaxel vehicle i.p., and on day 10 received a single s.c. injection of compound 63 or saline. The threshold force for hind paw withdrawal was recorded in each animal 3 days before (PRE) and 10 days after the first injection of paclitaxel vehicle. On that day, paw withdrawal latency was recorded immediately before (time 0) and at several times (30, 60, 90, 120 and 180 min) after the injection of compound 63 or saline. As expected, none the groups of animals treated with paclitaxel-vehicle developed mechanical allodynia (i.e. they did not show a decrease in the threshold force necessary to induce hind paw withdrawal) when evaluated 10 days after the first dose of paclitaxel-vehicle was administered (Fig. 10). A single s.c. injection of compound 63 (64 mg/kg, s.c.) on day 10 after the first injection of paclitaxel-vehicle did not affect the mechanical response of these control animals (Fig. 10).

### J) Effect of paclitaxel and paclitaxel-vehicle in wild type and sigma-1 receptor knockout mice.

Animals were treated once daily from days 1 to 5 with paclitaxel (2 mg/kg) or its vehicle i.p. The threshold force for hind paw withdrawal in the Dynamic Plantar Aesthesiometer was recorded 3 days before (PRE) and on several days after the first injection of paclitaxel or its vehicle. The threshold force for paw withdrawal before (PRE) the treatment with paclitaxel was very similar in wild type and sigma-1 knockout mice (Fig. 11).

The threshold force for paw withdrawal was significantly reduced in wild-type mice from days 7 to 10 after the first dose of paclitaxel (Fig. 11). In contrast, the threshold force for paw withdrawal was not significantly changed by paclitaxel treatment in σ1 receptor knockout mice (Fig. 11). Therefore, the animals devoid of σ1 receptor did not express and/or did not develop paclitaxel-induced mechanical allodynia.

The mechanical threshold of wild type and σ1 receptor knockout mice was not significantly different before (PRE) and after paclitaxel-vehicle administration (Fig. 12). These data indicate that σ1 receptor knockout mice are able to respond normally to a mechanical punctate stimulus.

### Example 3. Assessment of the preventive and curative antiallodynic effects of compound 63 in a chronic model of oxaliplatin-induced neuropathy in rats.

### Common procedures

Sixty male Sprague-Dawley rats (CERJ, France), weighing 136-169 g at the beginning of the experimental phase (first administration of compounds on D-2), were used. Rats were housed in a temperature (19.5°C - 24.5°C) and relative humidity (45 % - 65 %) controlled room with a 12 h - light/dark cycle, with *ad libitum* access to standard pelleted laboratory chow and water throughout the study.

Animals were housed 3 or 4 per cage and a four-days acclimation period was observed before any testing. Each rat was identified by tail markings.

Distilled water was used as Oxaliplatin vehicle. 0.5% Hydroxypropylmethylcellulose (HPMC) was used as compound 63 vehicle.

Principal Data Processing Systems: SigmaStat software (version 3.5, SPSS Science Software, Erkrath GmbH)

Six groups of 10 rats each were included in this study:
- Group 1: 0.5% HPMC, i.p. (tested compound vehicle) / Distilled water, i.p. (Oxaliplatin vehicle) (n=10)
- Group 2: 0.5% HPMC, i.p. (tested compound vehicle) / Oxaliplatin 3mg/kg, i.p. (n=10)
- Group 3: Compound 63 40 mg/kg, i.p. (BID x 18) / Oxaliplatin 3 mg/kg, i.p. (n=10)
- Group 4: Compound 63 20 mg/kg, i.p. (daily x 8) / Oxaliplatin 3 mg/kg, i.p. (n=10)
- Group 5: Compound 63 40 mg/kg, i.p. (daily x 8) / Oxaliplatin 3 mg/kg, i.p. (n=10)
- Group 6: Compound 63 80 mg/kg, i.p. (daily x 8) / Oxaliplatin 3 mg/kg, i.p. (n=10)

0.5% HPMC, compound 63 and Oxaliplatin were intraperitoneally injected (10 ml/kg). All treatments were administered in a coded order when it is possible.

Compound 63 preparation was made freshly everyday and the preparation was used during the morning and the afternoon for the group treated twice a day. Between both administrations, the preparation was stored at +4°C.

Chronic peripheral neuropathy was induced by repeated intraperitoneal injections of Oxaliplatin (3 mg/kg, i.p.) 3 times a week at days (D) 0, 2, 4, 7, 9, 11 and 14 (7 injections,Total dose = 21 mg/kg, i.p.).

Animals were tested 2 times during treatment with compound 63 (i.e. D8 and D15) and once 24 h after completion of treatment with compound 63 (*i.e.* D16).

In order to assess the preventive effect of compound 63, the compound was intraperitoneally administered (10 ml/kg) from 2 days before the first intraperitoneal injection of Oxaliplatin 3 mg/kg (D-2) until the day after the last Oxaliplatin injection (D15).

In order to assess the therapeutic effect of compound 63, the compound was intraperitoneally administered (10 ml/kg) from the first testing day (D8) until the day after the last Oxaliplatin injection (D15).

Animals belonging to the Oxaliplatin-treated group were dosed daily with the compound 63 vehicle (0.5% HPMC, 10 ml/kg) from the first testing day (D8) until the day after the last Oxaliplatin injection (D15).

During the daily treatment, compound 63 and 0.5% HPMC were administered during the morning whereas Oxaliplatin was administered on the afternoon.

Regarding the animal group treated twice a day with the compound 63, the time spent between both administrations was 8 hours. During the day of Oxaliplatin treatment, animals were dosed with the compound 63 30 min before Oxaliplatin injection.

During the testing days (*i.e.* D8, D15), compound 63 and 0.5% HPMC were administered 30 min before the test.

Cold allodynia was assessed by measuring the responses to thermal non-nociceptive stimulation (acetone test) on D8, D15 and D16.

Cold allodynia was assessed using the acetone test. In this test, latency of hindpaw withdrawal was measured after application of a drop of acetone to the plantar surface of both hindpaws (reaction time) and the intensity of the response was scored (cold score).

Reaction time to the cooling effect of acetone was measured within 20 sec (cut-off) after acetone application. Responses to acetone were also graded to the following 4-point scale: 0 (no response); 1 (quick withdrawal, flick of the paw); 2 (prolonged withdrawal or marked flicking of the paw); 3 (repeated flicking of the paw with licking or biting).

For each experimental group, the results were expressed as:
- The **reaction time** defined as the time expressed in sec required to elicit paw reaction (mean of 6 measures for each rat together ± SEM).
- The **cumulative cold score** defined as the sum of the 6 scores for each rat together ± SEM. The minimum score being 0 (no response to any of the 6 trials) and the maximum possible score being 18 (repeated flicking and licking or biting of paws on each of the six trials).

Statistical significance between the treated groups and the vehicle-treated group was determined by a Two-way Repeated Measures ANOVA on Ranks followed, when the F value is significant, by an appropriate *post-hoc* comparison (Bonferroni's test) using SigmaStat software. The significance level was p < 0.05. Statistical analyses were done using SigmaStat software.

### Results on oxaliplatin-induced cold allodynia

### A) Acetone test / Reaction time

As shown in Figures 13 and 14, Oxaliplatin induced cold allodynia as evidenced by the significant decrease in the reaction time of paw withdrawal after acetone application in the 0.5% HPMC/Oxaliplatin-treated group during the time course. This decrease was progressive and significant from D8 (- 19%, p<0.01) to D15 (- 37%, p<0.001) and D16 (- 36%, p<0.001) as compared to the HPMC/distilled water-treated group.

### A.1.) Preventive treatment with compound 63

Compound 63, intraperitoneally given twice a day at 40 mg/kg from D-2 to D15, induced a beneficial effect during the time course as shown in Figure 13. On D8, this effect was less marked but remained statistically present on D15 and on D16, 24 hours after completion of the treatment with compound 63.

### A.2.) Curative treatment with compound 63

Animals were dosed the first time with compound 63 at 20 mg/kg, 40 mg/kg and 80 mg/kg intraperitoneally on D8, during the first testing day and then, they received the treatment daily until D15 (last administration). In these experimental conditions (Figure 14), a dose-related effect was observed. Indeed, the lowest dose (*i.e.* 20 mg/kg, i.p.) induced a low effect throughout the study. However, the middle dose (*i.e.* 40 mg/kg, i.p.) induced an intermediate effect throughout the study.

Regarding the highest dose (*i.e.* 80 mg/kg, i.p.), a highly significant increase in the reaction time of paw withdrawal was observed as compared to the 0.5% HPMC / Oxaliplatin-treated group (19.1 ± 0.3 sec vs 14.7 ± 0.9 sec, p < 0.001). The beneficial effect observed on D8 remained statistically present on D15 (17.5 ± 0.5 sec vs 11.5 ± 1.6 sec, p < 0.01) and on D16 (15.5 ± 1.0 sec *vs* 11.5 ± 0.9 sec, p < 0.05).

### B) Acetone test / Cumulative cold score

On D8, as shown in Figures 15 and 16, Oxaliplatin induced a dramatic increase in the cumulative cold score, highly correlated with the decrease in the reaction time of paw withdrawal. Due to the continuation of the Oxaliplatin treatment, the cumulative cold score reached a maximum effect on D15 (7.0 ± 1.2 vs 1.4 ± 0.4, p<0.001) and remained statistically significant on D16 (6.7 ± 0.9 vs 1.3 ± 0.4, p<0.001).

### B.1.) Preventive treatment with compound 63

As observed in the reaction time of paw withdrawal, compound 63, intraperitoneally injected twice a day from D-2 to D15 at 40 mg/kg, induced a beneficial effect with values of cumulative cold score (2.3 ± 0.6 on D8; 3.0 ± 0.6 on D15 and 3.5 ± 0.7 on D16) closer to the values observed among the HPMC/distilled water-treated group than those obtained with the HPMC / Oxaliplatin-treated group (Figure 15).

### B.2.) Curative treatment with compound 63

Compound 63, intraperitoneally administered at 20 mg/kg, 40 mg/kg and 80 mg/kg from D8 to D15, also induced a dose-related effect in the cumulative cold score (Figure 16).

On D8, whereas the lowest dose of compound 63 (*i.e.* 20 mg/kg, i.p.) exhibited a low effect (4.3 ± 0.6), the highest dose (*i.e.* 80 mg/kg, i.p.) induced a highly significant decrease in the cumulative cold score (1.0 ± 0.4 vs 5.0 ± 0.8, p<0.001 as compared to the 0.5% HPMC / Oxaliplatin-treated group). On D15, the antiallodynic effect is maintained at the highest dose (1.8 ± 0.3, p<0.001) and only decreased on D16, 24 hours after the last injection of compound 63 (3.1 ± 0.6, p < 0.05).

Regarding the intermediate dose (*i.e.* 40 mg/kg, i.p.), it induced an intermediate effect with values of cumulative cold score bounded by those obtained with the HPMC/distilled water-treated group and those obtained with the HPMC / Oxaliplatin-treated group, as observed in the reaction time of paw withdrawal (Figure 16).

### Example 4. Assessment of the preventive and curative antiallodynic effects of compound 63 in a chronic model of cisplatin-induced neuropathy in rats.

### Common procedures

Male Wistar rats (250-300 g) were obtained from Harlan-Iberica (Barcelona, Spain).Upon arrival to our laboratory, animals were stored in standard transparent cages (40 × 28 × 25 cm) in environmentally controlled conditions (temperature=20 °C; humidity= 60%) with a 12 h light/12 h dark cycle (lights on between 08:00 and 20:00 h). Animals were allowed free access to tap water and standard laboratory rat chow (Harlan-Iberica, Barcelona, Spain).

Prior to the experiment, rats were habituated to isolation. Rats received intraperitoneal injections one day per week for 5 weeks of cisplatin (1 mg/kg) + vehicle (control group for acute treatment) or cisplatin (1 mg/kg) + compound 63 (25 mg/kg). The last week (day 36) control group received an acute administration of 64 mg/kg of compound 63.

Mechanical allodynia was assessed before cisplatin administration (basal) and at the end of each experimental week (w1, w2, w3, w4). For the acute evaluation of compound 63 (w5), the mechanical allodynia was assessed 50 minutes after drug administration. On those days, rats were placed individually on an elevated iron mesh in a clear plastic cage and were allowed to adapt to the testing environment for at least 10 min. Habituation to this environment was also performed on the two days before assessment. Calibrated von Frey Hairs (0.9, 1.4, 2.1, 2.5, 3, 4, 5.5, 7.5, 8, 10.5, 13, 14, 15, 17, 25, 27, 32 and 40 g) were applied to the plantar aspect of each hindpaw, from below the mesh floor. Each stimulus was applied for approximately 1 s with an interstimulus interval of approximately 3 s. Only robust and immediate withdrawal responses from the stimulus were recorded. A positive result was considered when 3 or more withdrawal responses were obtained from 5 consecutive trials with each monofilament. Mechanical allodynia was defined as a significant decrease in von Frey hair threshold evoked by mechanical stimuli.

### Results on cisplatin-induced mechanical allodynia

### Acute treatment of compound 63

As expected, cisplatin administered once a week induced a progressive significant reduction in von Frey pressure response in the control group that was clear at weeks 3 and 4. Acute administration of 64 mg/kg of compound 63 on week 5 reverted the effect induced by cisplatin to basal levels (Fig. 17).

### Preventive treatment with compound 63

Cisplatin administered once a week for 5 weeks induced a reduction in von Frey pressure response. After last cisplatin administraton, on week 5, this parameter recovered basal levels at week 8. Interestingly, coadministration of 25 mg/kg of compound 63 twice a day, one day per week, the same days that cisplatin is administered, prevented the development of mechanical allodynia by cisplatin. The present results show that compound 63 prevents the development of mechanical allodynia induced by cisplatin in a rat model of peripheral neuropathy (Fig. 18).

## Claims

1. A combination of at least one sigma ligand and at least one chemotherapeutic drug for simultaneous, separate or sequential administration, wherein the sigma ligand has the general formula (I): 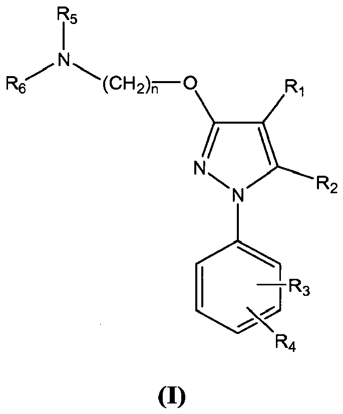 wherein
**R₁** is selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic heterocyclyl, substituted or unsubstituted aromatic heterocyclyl, substituted or unsubstituted heterocyclylallcyl, -COR₈, -C(O)OR₈,-C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, and halogen;
**R₂** is selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, and halogen;
**R₃** and **R₄** are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈,-C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, and halogen, or together they form a optionally substituted fused ring system;
**R₅** and **R₆** are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈,-C(O)NR₈R₉, -CH=NR₈, -CN, -OR₈, -OC(O)R₈, -S(O)ₜ-R₈ , -NR₈R₉, -NR₈C(O)R₉, -NO₂, -N=CR₈R₉, and halogen, or together form, with the nitrogen atom to which they are attached, a substituted or unsubstituted heterocyclyl group;
**n** is selected from 1, 2, 3, 4, 5, 6, 7 and 8;
**t** is 1,2 or 3;
**R₈** and **R₉** are each independently selected from hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer or solvate thereof;
and
the chemotherapeutic drug is selected from the group formed by taxanes, vinca alkaloids, drugs derived from platinum and thalidomide.

2. A combination according to claim 1, wherein R₁ is selected from H, -COR₈ and substituted or unsubstituted alkyl.

3. A combination according to claims 1 or 2, wherein R₂ is H or alkyl.

4. A combination according to any preceding claims, wherein R₃ and R₄ together form a fused naphthyl ring system.

5. A combination according to any preceding claims, wherein R₅ and R₆ together form a morpholine-4-yl group.

6. A combination according to any preceding claims, wherein the sigma ligand of formula (I) is 4-{2-[5-Methyl-1-(naphthalen-2-yl)-1H-pyrazol-3-yloxy]ethyl} morpholine, or a pharmaceutically acceptable salt, isomer or solvate thereof.

7. A combination according to any preceding claims, wherein the sigma ligand of formula (I) is 4-{2-[5-Methyl-1-(naphthalen-2-yl)-1H-pyrazol-3-yloxy]ethyl} morpholine hydrochloride.

8. A combination according to any preceding claims, wherein the chemotherapeutic drug is selected from the group formed by paclitaxel, oxaliplatin, cisplatin, vincristine, and thalidomide.

9. A combination according to any preceding claims, wherein the combination comprises 4- {2-[5-Methyl-1-(naphthalen-2-yl)-1H-pyrazol-3-yloxy]ethyl} morpholine and a chemotherapeutic drug selected from paclitaxel, oxaliplatin or cisplatin.

10. A combination as defined in any of claims 1 to 9 for its use in the manufacture of a medicament.

11. A combination as defined in any of claims 1 to 9 for its use in the prevention or treatment of pain induced by chemotherapy.

12. A combination according to claim 11, wherein the pain is selected from peripheral neuropathic pain, allodynia, causalgia, hyperalgesia, hyperesthesia, hyperpathia, neuralgia, neuritis and neuropathy.

13. A compound of formula (I) as defined in any of claims 1 to 7, or a pharmaceutically acceptable salt, isomer or solvate thereof, for its use in the treatment or prevention of pain induced by chemotherapy, wherein the chemotherapeutic drug is selected from the group formed by taxanes, vinca alkaloids, drugs derived from platininum and thalidomide.

14. A compound according to claim 13, or a pharmaceutically acceptable salt, isomer or solvate thereof, for its use in the prevention of pain induced by chemotherapy, wherein the chemotherapeutic drug is selected from the group formed by taxanes, vinca alkaloids, drugs derived from platininum and thalidomide.

15. A compound according to claims 13 or 14, wherein the pain is selected from peripheral neuropathic pain, allodynia, causalgia, hyperalgesia, hyperesthesia, hyperpathia, neuralgia, neuritis and neuropathy.

## Patentansprüche

1. Kombination aus mindestens einem Sigma-Liganden und mindestens einem Chemotherapeutikum für die gleichzeitige, separate oder sequentielle Verabreichung, wobei der Sigma-Ligand die allgemeine Formel (I) aufweist 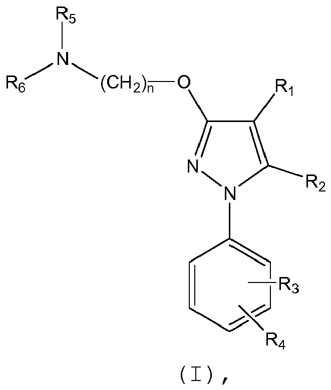 in der
**R₁** aus der Gruppe bestehend aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Arylalkyl, substituiertem oder unsubstituiertem, nicht-aromatischem Heterocyclyl, substituiertem oder unsubstituiertem, aromatischem Heterocyclyl, substituiertem oder unsubstituiertem Heterocyclylalkyl, -COR₈, -C(O)OR₈, -C(O)NR₈R₉, -CH=NR₈, -CN, -OR₈,-OC(O)R₈, -S(O)ₜ-R₈, -NR₈R₉, -NR₈C(O)R₉, -NO₂, -N=CR₈R₉ und Halogen ausgewählt ist;
**R₂** aus der Gruppe bestehend aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Arylalkyl, substituiertem oder unsubstituiertem, aromatischem oder nicht-aromatischem Heterocyclyl, substituiertem oder unsubstituiertem Heterocyclylalkyl, -COR₈, - C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉ und Halogen ausgewählt ist;
**R₃** und **R₄** unabhängig aus der Gruppe bestehend aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Arylalkyl, substituiertem oder unsubstituiertem, aromatischem oder nicht-aromatischem Heterocyclyl, substituiertem oder unsubstituiertem Heterocyclylalkyl, -COR₈, - C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉ und Halogen ausgewählt sind oder zusammen ein kondensiertes Ringsystem bilden, welches wahlweise substituiert ist;
**R₅** und **R₆** unabhängig aus der Gruppe bestehend aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Arylalkyl, substituiertem oder unsubstituiertem, aromatischem oder nicht-aromatischem Heterocyclyl, substituiertem oder unsubstituiertem Heterocyclylalkyl, -COR₈,-C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉ und Halogen ausgewählt sind oder zusammen mit dem Stickstoffatom, an welches sie gebunden sind, eine substituierte oder unsubstituierte Heterocyclylgruppe bilden;
n aus 1, 2, 3, 4, 5, 6, 7 und 8 ausgewählt ist;
t 1, 2 oder 3 ist;
**R₈** und **R₉** jeweils unabhängig aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem, aromatischem oder nicht-aromatischem Heterocyclyl, substituiertem oder unsubstituiertem Alkoxy, substituiertem oder unsubstituiertem Aryloxy und Halogen ausgewählt sind;
oder ein pharmazeutisch annehmbares Salz, Isomer oder Solvat davon;
wobei das Chemotherapeutikum aus der Gruppe bestehend aus Taxanen, Vincaalkaloiden, von Platin und von Thalidomid abgeleiteten Arzneimitteln ausgewählt ist.

2. Kombination nach Anspruch 1, wobei R₁ aus H, -COR₈ und substituiertem und unsubstituiertem Alkyl ausgewählt ist.

3. Kombination nach den Ansprüchen 1 oder 2, wobei R₂ H oder Alkyl ist.

4. Kombination nach einem der vorhergehenden Ansprüche, wobei R₃ und R₄ zusammen ein kondensiertes Naphthyl-Ringsystem bilden.

5. Kombination nach einem der vorhergehenden Ansprüche, wobei R₅ und R₆ zusammen eine Morpholin-4-yl-Gruppe bilden.

6. Kombination nach einem der vorhergehenden Ansprüche, wobei der Sigma-Ligand der Formel (I) 4-{2-[5-Methyl-1-(naphthalin-2-yl)-1H-pyrazol-3-yloxy]ethyl}morpholin oder ein pharmazeutisch annehmbares Salz, Isomer oder Solvat davon ist.

7. Kombination nach einem der vorhergehenden Ansprüche, wobei der Sigma-Ligand der Formel (I) 4-{2-[5-Methyl-1-(naphthalin-2-yl)-1H-pyrazol-3-yloxy]ethyl}morpholinhydrochlorid ist.

8. Kombination nach einem der vorhergehenden Ansprüche, wobei das Chemotherapeutikum aus der Gruppe bestehend aus Paclitaxel, Oxaliplatin, Cisplatin, Vincristin und Thalidomid ausgewählt ist.

9. Kombination nach einem der vorhergehenden Ansprüche, wobei die Kombination 4-{2-[5-Methyl-1-(naphthalin-2-yl)-1H-pyrazol-3-yloxy]ethyl}morpholin und ein Chemotherapeutikum ausgewählt aus Paclitaxel, Oxaliplatin oder Cisplatin umfasst.

10. Kombination wie in einem der Ansprüche 1 bis 9 definiert zur Verwendung in der Herstellung eines Arzneimittels.

11. Kombination wie in einem der Ansprüche 1 bis 9 definiert zur Verwendung in der Vorbeugung oder Behandlung von Schmerzen, die durch Chemotherapie hervorgerufen sind.

12. Kombination nach Anspruch 11, wobei der Schmerz aus peripheren neuropathischen Schmerzen, Allodynie, Kausalgie, Hyperalgesie, Hyperästhesie, Hyperpathie, Neuralgie, Neuritis und Neuropathie ausgewählt ist.

13. Verbindung der Formel (I) wie in einem der Ansprüche 1 bis 7 definiert oder ein pharmazeutisch annehmbares Salz, Isomer oder Solvat davon zur Verwendung in der Behandlung oder Vorbeugung von Schmerzen, die durch Chemotherapie hervorgerufen sind, wobei das Chemotherapeutikum aus der Gruppe bestehend aus Taxanen, Vincaalkaloiden, von Platin und von Thalidomid abgeleiteten Arzneimitteln ausgewählt ist.

14. Verbindung nach Anspruch 13 oder ein pharmazeutisch annehmbares Salz, Isomer oder Solvat davon zur Verwendung in der Vorbeugung von Schmerzen, die durch Chemotherapie hervorgerufen sind, wobei das Chemotherapeutikum aus der Gruppe bestehend aus Taxanen, Vincaalkaloiden, von Platin und von Thalidomid abgeleiteten Arzneimitteln ausgewählt ist.

15. Verbindung nach den Ansprüchen 13 oder 14, wobei der Schmerz aus peripheren neuropathischen Schmerzen, Allodynie, Kausalgie, Hyperalgesie, Hyperästhesie, Hyperpathie, Neuralgie, Neuritis und Neuropathie ausgewählt ist.

## Revendications

1. Combinaison d'au moins un ligand sigma et d'au moins un médicament chimiothérapeutique pour une administration simultanée, séparée ou séquentielle, dans laquelle le ligand sigma a la formule générale (I) : 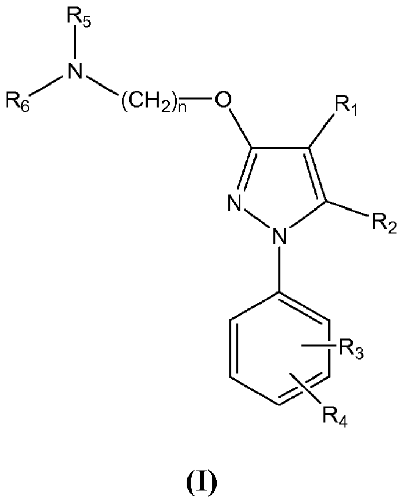 dans laquelle
R₁ est choisi dans le groupe constitué de l'hydrogène, alkyle substitué ou non substitué, cycloalkyle substitué ou non substitué, alcényle substitué ou non substitué, aryle substitué ou non substitué, arylalkyle substitué ou non substitué, hétérocyclyle non aromatique substitué ou non substitué, hétérocyclyle aromatique substitué ou non substitué, hétérocyclylalkyle substitué ou non substitué, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, et halogène ;
R₂ est choisi dans le groupe constitué de l'hydrogène, alkyle substitué ou non substitué, cycloalkyle substitué ou non substitué, alcényle substitué ou non substitué, aryle substitué ou non substitué, arylalkyle substitué ou non substitué, hétérocyclyle aromatique ou non aromatique substitué ou non substitué, hétérocyclylalkyle substitué ou non substitué, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, et halogène ;
R₃ et R₄ sont choisis indépendamment dans le groupe constitué de l'hydrogène, alkyle substitué ou non substitué, cycloalkyle substitué ou non substitué, alcényle substitué ou non substitué, aryle substitué ou non substitué, arylalkyle substitué ou non substitué, hétérocyclyle aromatique ou non aromatique substitué ou non substitué, hétérocyclylalkyle substitué ou non substitué, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, et halogène, ou conjointement ils forment un système cyclique condensé éventuellement substitué ;
R₅ et R₆ sont choisis indépendamment dans le groupe constitué de l'hydrogène, alkyle substitué ou non substitué, cycloalkyle substitué ou non substitué, alcényle substitué ou non substitué, aryle substitué ou non substitué, arylalkyle substitué ou non substitué, hétérocyclyle aromatique ou non aromatique substitué ou non substitué, hétérocyclylalkyle substitué ou non substitué, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, et halogène, ou conjointement ils forment, avec l'atome d'azote auquel ils sont fixés, un groupe hétérocyclyle substitué ou non substitué ;
n est choisi parmi 1, 2, 3, 4, 5, 6, 7 et 8 ;
t vaut 1, 2 ou 3 ;
R₈ et R₉ sont chacun choisis indépendamment dans l'hydrogène, alkyle substitué ou non substitué, cycloalkyle substitué ou non substitué, alcényle substitué ou non substitué, aryle substitué ou non substitué, hétérocyclyle aromatique ou non aromatique substitué ou non substitué, alcoxy substitué ou non substitué, aryloxy substitué ou non substitué, et halogène;
ou un sel, un isomère ou un solvate pharmaceutiquement acceptable de celui-ci ;
et le médicament chimiothérapeutique est choisi dans le group constitué des taxanes, des alcaloïdes vinca, des médicaments dérivés du platine et de la thalidomide.

2. Combinaison selon la revendication 1, dans laquelle R₁ est choisi parmi H, -COR₈ et alkyle substitué ou non substitué.

3. Combinaison selon la revendication 1 ou 2, dans laquelle R₂ est H ou alkyle.

4. Combinaison selon l'une quelconque des revendications précédentes, dans laquelle R₃ et R₄ forment conjointement un système cyclique naphtyle condensé.

5. Combinaison selon l'une quelconque des revendications précédentes, dans laquelle R₅ et R₆ forment conjointement un groupe morpholine-4-yle.

6. Combinaison selon l'une quelconque des revendications précédentes, dans laquelle le ligand sigma de formule (I) est la 4-{2-[5-méthyl-1-(naphtalèn-2-yl)-1H-pyrazol-3-yloxy]éthyl}-morpholine, ou un sel, un isomère ou un solvate pharmaceutiquement acceptable de celle-ci.

7. Combinaison selon l'une quelconque des revendications précédentes, dans laquelle le ligand sigma de formule (I) est le chlorhydrate de 4-{2-[5-méthyl-1-(naphtalèn-2-yl)-1H-pyrazol-3-yloxy]éthyl}-morpholine.

8. Combinaison selon l'une quelconque des revendications précédentes, dans laquelle le médicament chimiothérapeutique est choisi dans le groupe constitué du paclitaxel, de l'oxaliplatine, du cisplatine, de la vincristine et de la thalidomide.

9. Combinaison selon l'une quelconque des revendications précédentes, dans laquelle la combinaison comprend de la 4-{2-[5-méthyl-1-(naphtalèn-2-yl)-1H-pyrazol-3-yloxy]éthyl}-morpholine et un médicament chimiothérapeutique choisi parmi le paclitaxel, l'oxaliplatine ou le cisplatine.

10. Combinaison telle que définie dans l'une quelconque des revendications 1 à 9, pour son utilisation dans la fabrication d'un médicament.

11. Combinaison telle que définie dans l'une quelconque des revendications 1 à 9, pour son utilisation dans la prévention ou le traitement d'une douleur induite par la chimiothérapie.

12. Combinaison selon la revendication 11, dans laquelle la douleur est choisie parmi la douleur neuropathique périphérique, l'allodynie, la causalgie, l'hyperalgésie, l'hyperesthésie, l'hyperpathie, la névralgie, la neurite et la neuropathie.

13. Composé de formule (I) tel que défini dans l'une quelconque des revendications 1 à 7, ou sel, isomère ou solvate pharmaceutiquement acceptable de celui-ci, pour son utilisation dans le traitement ou la prévention d'une douleur induite par la chimiothérapie, dans lequel le médicament chimiothérapeutique est choisi dans le group constitué des taxanes, des alcaloïdes vinca, des médicaments dérivés du platine et de la thalidomide.

14. Composé selon la revendication 13, ou sel, isomère ou solvate pharmaceutiquement acceptable de celui-ci, pour son utilisation dans la prévention d'une douleur induite par la chimiothérapie, dans lequel le médicament chimiothérapeutique est choisi dans le group constitué des taxanes, des alcaloïdes vinca, des médicaments dérivés du platine et de la thalidomide.

15. Composé selon la revendication 13 ou 14, dans lequel la douleur est choisie parmi la douleur neuropathique périphérique, l'allodynie, la causalgie, l'hyperalgésie, l'hyperesthésie, l'hyperpathie, la névralgie, la neurite et la neuropathie.
